(19) Europäisches Patentamt
European Patent Office
Office européen des brevets

(11) EP 0 776 160 B1

(12) EUROPEAN PATENT SPECIFICATION

(45) Date of publication and mention of the grant of the patent:
**08.03.2000 Bulletin 2000/10**

(21) Application number: **95944559.4**

(22) Date of filing: **29.12.1995**

(51) Int Cl.[7]: **A01N 35/02**
// (A01N35/02, 65:00, 25:30)

(86) International application number:
**PCT/US95/17009**

(87) International publication number:
**WO 96/41528 (27.12.1996 Gazette 1996/56)**

(54) **USE OF SAPONIN FOR PATHOGEN CONTROL**

VERWENDUNG VON SAPONIN ALS SYNERGIST FÜR PESTIZIDE FLAVONOIDALDEHYDE

UTILISATION DE SAPONINE DANS LA LUTTE CONTRE LES GERMES PATHOGENES

(84) Designated Contracting States:
**AT BE CH DE DK ES FR GB GR IE IT LI LU MC NL PT SE**

(30) Priority: **07.06.1995 US 476840**

(43) Date of publication of application:
**04.06.1997 Bulletin 1997/23**

(73) Proprietor: **PROGUARD, INC.**
**Suisun City, CA 94585 (US)**

(72) Inventors:
- **EMERSON, Ralph, W.**
**Davis, CA 95616 (US)**
- **CRANDALL, Bradford, G., Jr.**
**Davis, CA 95616 (US)**

(74) Representative:
**Smulders, Theodorus A.H.J., Ir. et al**
**Vereenigde Octrooibureaux**
**Nieuwe Parklaan 97**
**2587 BN 's-Gravenhage (NL)**

(56) References cited:
**WO-A-94/27434**
**FR-A- 2 529 755**
**FR-A- 2 697 133**
**US-A- 2 465 854**
**US-A- 4 978 686**

- **PATENT ABSTRACTS OF JAPAN vol. 17, no. 513 (C-1111), 16 September 1993 & JP,A,05 139924 (ITOUEN), 8 June 1993,**
- **DATABASE WPI Week 9232 Derwent Publications Ltd., London, GB; AN 92-262127 [32] XP002004476 & JP,A,04 176 460 (TANAKA) , 24 June 1992**
- **D.E.H.FREAR: "Chemistry of Insecticides and Fungicides" , D.VAN NOSTRAND , NEW YOORK, US XP002004469 pages 184-191, "WETTING AND SPREADING AGENTS" see page 185, paragraph 4 - page 186, paragraph 1**
- **DATABASE WPI Week 9431 Derwent Publications Ltd., London, GB; AN 94-252673 [31] XP002004470 & JP,A,06 183 925 (TASHIRO) , 5 July 1994**
- **DATABASE WPI Week 9251 Derwent Publications Ltd., London, GB; AN 92-420381 [51] XP002004530 & JP,A,04 316 506 (NAKANO SUMESE) , 6 November 1992**
- **CHEMICAL ABSTRACTS, vol. 100, no. 17, 23 April 1984 Columbus, Ohio, US; abstract no. 134151, R.OHTSUKA ET AL.: "Effects of ABION CA chemicals on vegetable diseases" XP002004527 & KYUSHU BYOGAICHU KENKYUKAI HO, vol. 29, 1983, pages 48-51,**
- **CHEMICAL ABSTRACTS, vol. 120, no. 9, 28 February 1994 Columbus, Ohio, US; abstract no. 99242, S.HAGIWARA ET AL.: "Effect of cinnamaldehyde on the growth of Rhizoctania solani (AG2-2IIIB) and development of brown patch disease on bentgrass" XP002004528 & HOKKAIDO SOCHI KENKYUKAIHO, vol. 27, 1993, pages 74-77,**
- **DATABASE WPI Week 9324 Derwent Publications Ltd., London, GB; AN 93-191413 [24] XP002004531 & JP,A,05 117 125 (DAINICHISEIKA COLOR & CHEM) , 14 May 1993**
- **DATABASE WPI Week 8235 Derwent Publications Ltd., London, GB; AN 82-73641E [35] XP002004471 & JP,A,57 120 501 (SAOTOME) , 27 July 1982**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- **DATABASE WPI Week 8948 Derwent Publications Ltd., London, GB; AN 89-351364 [48] XP002004474 & JP,A,01 261 303 (TAIYO KORYO) , 18 October 1989**
- **CHEMICAL ABSTRACTS, vol. 109, no. 1, 4 July 1988 Columbus, Ohio, US; abstract no. 2423, N.ISHIBASHI ET AL.: "Nematicidal effect of cinnamic aldehyde on root-knot nematode, Meloidogyne incognita" XP002004529 & KYUSHU BYOGAICHU KENKYUKAIHO, vol. 33, 1987, pages 122-125,**

## Description

## INTRODUCTION

### Field of the Invention

The invention relates to the use of saponin as a synergist in methods and formulations for controlling the colonization and/or growth of plant and animal pathogens on a material. The invention is exemplified by the use of compositions comprising saponin in combination with cinnamic aldehyde, α-hexyl cinnamic aldehyde, and/or coniferyl aldehyde to control growth of fungi and parasitic insects which colonize the surfaces of plant parts and tissues.

### Background of the Invention

Pesticides and fungicides are normally designed to treat or rid a host plant or material of an infesting organism. The pesticides and fungicides used generally are applied to those surfaces of a plant or plant part that are colonized by the organisms. The colonizing organisms include sap-sucking insects and pathogenic fungi; both groups are capable of inflicting severe damage to the host plant, including stunting the growth of the host plant and decreasing plant productivity, to killing the host plant.

Pathogenic insects which infest plants include those insect species which are symbiotic with bacteria, such as aphids, leaf hoppers, and white fly; the host insect cannot survive without the symbionts. As an example, aphids *(homoptera)* possess symbiotic bacteria of the genus *Buchnera* in cells called mycetocytes within the hemocoel. The bacteria are transmitted directly from the maternal aphid to her offspring and aposymbiotic aphids do not occur naturally. The bacteria may provide lipids which are required for embryogenesis of the host insect but which are absent or in low concentrations in phloem sap in plants infected by the insects.

The plant pathogens also include the grape phylloxera *(Daktulosphaira vitifoliae),* an aphid-like insect, and nematodes. Phylloxera is native to the United States east of the Rocky Mountains, where it lives on native wild species of grapes, which have evolved resistance to the feeding of the insect. The European grape *(Vitis vinifera*), which is used to produce wine, evolved in western Asia and has no resistance to phylloxera. Stem and bulb nematode (*Ditylenchus dipsaci*) has been reported in all the major agricultural regions in California. This wide distribution probably reflects its spread on infested planting material such as garlic cloves. Wherever such infested material is grown, the nematode may be introduced. *Ditylenchus dipsaci* can be found parasitizing a wide range of cultivated and wild plants. Nematodes produce galls in infected tissue. In addition to the disturbance caused to plants by the nematode galls themselves, damage to infected plants is increased by certain parasitic fungi, which can easily attach to the weakened root tissues and the hypertrophid, undifferentiated cells of the galls. Moreover, some fungi, for example, *Pythium, Fusarium,* and *Rhizoctonia*, grow and reproduce much faster in the galls than in other areas of the root, thus inducing an earlier breakdown of the root tissues.

Fungi pathogenic for plants occur in most groups of fungi. A few, such as rusts (*Uredinales, Phragmidrium*), powdery mildew (*Erysiphacea, Sphaerotheca*) and downy mildew (*Peronosporacea*) are obligate parasites associated with specific host plants which elaborate nutrients required by the pathogen. Additionally, fungi from the genera *Aspergillus, Alternaria, Fusarium,* and *Penicillium* can contaminate food crops and their products, all of which fungi are known to produce mycotoxins such as aflatoxins, fumonisins, fusaric acid, TA/AAL toxins, zearalenone, and trichothecene, 5-butylpicolinic acid and related phytotoxic pyridine derivatives. These mycotoxins are highly toxic to a variety of species including plants and humans and can be found in commercially prepared food stuffs including milk and milk products, beans, cereals, coconuts, peanuts, sweet potatoes and commercially prepared animal feeds.

A variety of pesticide and fungicide compositions are used for controlling plant pathogens. For example, protective fungicidal sprays on a 6-7 day schedule for both rust and powdery mildew when environmental conditions favor disease development are the typical means of control. Two frequently used systemic fungicides are benomyl and triforine. The older fungicides also include inorganic compounds such as copper and sulphur and the organic protectants such as thiram, captom, mameb, and chlorotholonil. These compounds act only at the surface of the plant and must be present at or before appearance of the fungal pathogen in order to prevent infection. These older fungicides are multisite inhibitors, *i.e.*, they affect many metabolic activities in a fungus.

The newer fungicides tend to be highly effective organic systemics such as benzimidazoles, sterol biosynthesis inhibitors, carboxanilides, and phenylamides which act internally as well as at the plant surface. In contrast to the older surface protectants, the systemic fungicides are generally effective at much lower dosages and can cure established fungal infections, a critical factor in disease management. The systemic fungicides usually act at a single target site in the fungus, interfering with specific metabolic processes that are necessary for production of all new cell material required for growth, maintenance, and virulence of the fungal organism. These preparations typically are effective only against fungal pathogens.

The widespread use of pesticides, fungicides and chemical preservatives has resulted in the development and evolution of resistant pathogens. As environmental and health care concerns continue to mount, it will be necessary to identify and/or develop new pesticides and fungicides to meet the environmental standards of the future, particularly those which are natural products for consumption by animals and thus have lower animal and environmental toxicities. It therefore is of interest to develop new methods and formulations are needed to control plant and animal pathogens and to decrease the level of toxic metabolites present in consumable products and in the environment in general elaborated by the pathogens. Saponins are a type of sterol glycoside widely distributed in plants. The saponins have diverse biological activities finding use in agents employed as fungicides, insecticides, anticancer agents, cosmetics, food preservatives and fertilizers with growth-promoting and insecticidal effects. Saponin also is used to remove cholesterol from dairy products and as a feed supplement for livestock, such as chickens, to reduce cholesterol levels in eggs and reduce manure odor. Despite these many and diverse uses for saponin, the combined effect of saponin with other agents having pesticidal and/or fungicidal activities remains largely unexplored.

Relevant Literature

The use of saponin from agave in compositions to protect humans and other animals against pests such as mosquitoes and ticks is described in British patent application no. 9203522. A fertilizer with growth-promoting and insecticidal effects which contains saponin, lignin, phosphorous, nitrogen, potassium, and rare earth metals is disclosed in Canadian patent application no. 90100605. A food preservative containing saponin from aloe woods and a *p*-hydroxy benzoic acid ester is disclosed in Japanese patent application no. 61065802.
The use of compositions containing saponin from *Yucca schidigera* or *Y. Hedera helix* against non-aquatic mollusks such as snails and slugs is disclosed in U.S. Patent No. 5,290,557. The effect of saponin on grasshopper nymphs is disclosed by Westcott *et al.* (1992) *Ann. Entomol. Soc. Am.* 85(3):304-309. An antifungal drug containing saponin extracts from asparagus is disclosed in Japanese patent application no. 2157205. The antimicrobial properties of agents comprising one or more pressed juices from a variety of plants and/or solvent extracts or their condensates in acetic acid compositions are disclosed in Japanese patent application no. 91106370. The antimicrobial and pest activity of a saponin isolated from fruit pulp, tobacco, and seed are described by Okunji *et al., (Int. J. Crude Drug Res.,* (1990) 28(3):193-199), Gruenweller *et al., (Phytochemistry* (Oxf), (1990) 29(8):2485-2490), and by Lalithat *et al.,* (*Int. Pest Control* (1988) 30(2):42-45), respectively. Use of a saponin from camellia leaves for the control of anthrose, rice blast, and rice *helminthosporium* leaf spot is described in Japanese patent application no. 61007290. The antimicrobial properties of saponins are also reported in German patent application no. 3724595.
U.S. Patent No. 2,465,854 describes an insecticidal composition containing a cinnamic aldehyde derivative. Control of *Verticillium* using cinnamaldehyde in the substrate in which mushrooms are grown is disclosed in U.S. Patent No. 5,149,715. U.S. Patent No. 4,402,950 describes the deactivation of viruses inside living human and animal organisms by application of a terpene obtainable from aromatic plants by steam application. The terpenes cited are: black pepper oil, cinnamon flour oil, cardamon oil, linallyl acetate, cinnamic aldehyde, safrol, carvon and cis/trans citrao. U. S. Patent No. 4,477,361 describes a cinnamic compound containing an antimicrobial surfactant which is rendered substantive to the surface being washed.
The antibotulinal properties of various aromatic and aliphatic aldehyde are disclosed in Bowles and Miller (*J. Food Protection* (1993) 56:788-794). Other formulations which include cinnamic aldehyde have been reported to protect crops from attack by pathogenic microbes. *See* U.S. Patent Nos. 4,978,686 and 5,149,715 and French patent application no. 2529755.

SUMMARY OF THE INVENTION

The present invention relates to saponin containing formulations and methods for their use for controlling the colonization and/or growth of plant and animal pathogens on a material. Provided is a method for controlling pathogenic organisms on plants, as well as seeds and seedlings and for providing plants seeds and seedlings substantially free of plant pathogens. The method includes the step of contacting one or more parts or tissues of a diseased plant or a plant susceptible to attack by pathogens with a saponin-containing formulation in an amount sufficient to control growth of target pathogenic organisms. Also provided are methods and compositions containing saponin for controlling the level of toxic metabolites present in a variety of consumable products that are either colonized or capable of being colonized by toxin-producing microorganisms. These methods include the steps of contacting a consumable product or a precursor of such product with a saponin-containing antipathogenic and/or toxin reducing agent which limits the colonization of, kills or displaces one or more microorganisms which colonize the consumable material or precursor and which produce toxin(s). The invention finds use in treating agricultural crops for pathogenic organisms and controlling the level of toxic metabolites present in consumable products derived from plant materials, as well as decreasing contamination of the food chain by fungal toxins and toxic metabolites. The compositions used can include in addition

to saponin, aromatic aldehydes, such as cinnamic aldehyde, α-hexyl cinnamic aldehyde, and coniferyl aldehyde. Saponin formulations also find use in killing nematodes.

BRIEF DESCRIPTION OF THE PREFERRED EMBODIMENTS

Methods and formulations employing saponin as synergist in combination with a natural product, such as one or more aromatic aldehydes, are provided for substantially controlling the colonization and/or growth of plant and animal pathogens, such as fungi, insects, arachnids and non-amphibious/non-aquatic mollusks, on materials such as plants, plant parts and agricultural products that are capable of supporting growth or otherwise being colonized and infested by pathogens. Preferably the saponin acts as a synergist with the natural product. The compositions are applied to the plant either before it is harvested or to a plant or other material after harvest and/or processing. Most preferably, the composition is applied to the plant, plant part or tissue before harvest. The composition is preferably biodegradable and most preferably is provided as an aqueous solution or as an emulsion in a biodegradable water-soluble anhydrous non-ionic surfactant, such as Tween 80. The susceptibility of particular pathogen to the composition can be evaluated either *in vitro* or *in vivo.* By "synergist" is intended a compound which increases the effect of at least one other compound present in a formulation whereby the combined action is greater than the sum of their separate, individual actions. By "natural product" is intended an organic compound of natural origin that is unique to one organism, or common to a small number of closely related organisms, and includes secondary metabolites of fungi and chemicals produced by plants. As an example, a fungus and/or insect colonizing surface of a plant part such as a leaf, root, or flower part, or a tissue such as xylem or phloem, is contacted with a natural product to kill, displace or otherwise retard or eliminate the growth of pathogen. By "colonizing" is intended association of a microorganism or insect with a material such as a plant part or tissue from which the pathogen derives nutrients, typically essential nutrients such as amino acids, particularly methionine. By "growth" is intended an irreversible change in an organism accompanied by the utilization of material, and resulting in increased volume, dry weight or protein content and/or increase in population or colonization. By "pathogen" is intended an organism such as a fungus, bacterium, insect, arachnid, worm and non-amphibious/non-aquatic mollusks causing damage or disease to a biological host. By "material" is intended any substance capable of supporting colonization and/or growth of a target pathogen. The method to biocontrol pathogen infestations on plants is to use a formulation containing one or more saponins in combination with aromatic aldehydes, particularly using naturally occurring compounds such as the aromatic aldehydes cinnamic aldehyde, α-hexyl cinnamic aldehyde and coniferyl aldehyde. By "biocontrol" is intended control of plant pathogens via direct antipathogenic activity and/or induced resistance of the host plant to pathogen infestation.

The compositions and methods of the subject invention offer several advantages over existing compositions and methods. By identifying and exploiting the antipathogenic properties of saponin, the effective amount or concentration of one or more other formulation ingredients can be modified while decreasing phytotoxic effects and preserving or enhancing the antipathogenic effect of the formulation, particularly by allowing for a reduction in the concentration of one or more other ingredients in a given formulation. As an example, although an aromatic aldehyde, cinnamic aldehyde, has been reported to exhibit antifungal properties, it has not previously been used on plants in combination with saponin. A single application of a saponin emulsion containing cinnamic aldehyde is sufficient for long term protection of the plant host from pathogenic organisms, including both rust and powdery mildew, and is effective at lower concentrations than has been reported previously. The formulations of the subject invention also are effective against pests known to be resistant to conventional treatments, including such pests as thrips and melon aphid. Phytotoxicity of the formulation is also decreased due to the lower concentration of aldehyde which is used, the lesser number of applications required, and to the steroid moiety of the saponin. Thus, significant cost and environmental impact savings are realized. Furthermore, the long term control of pathogenic organisms results in a healthier plant and an improved yield of produce of the host plant as compared to untreated plants; the lower concentrations and single dose of antipathogenic agents decrease the likelihood of damage to the plant or its crop as well as decrease the likelihood of any adverse side effects to workers applying the pesticide, or to animals, fish or fowl which ingest the tissues or parts of treated plants.

The subject formulations also provide for effective control of both fungi and insects, eliminating the need for application of multiple agents. In particular situations, such as where an insect damages a plant part or tissue and a secondary fungal disease develops, this aspect of the invention is particularly advantageous. Another advantage is that contamination of consumable agricultural products can be prevented or significantly decreased to a level safe for consumption. Agricultural products can be treated either preharvest or postharvest. Moreover, by treating a plant in the field with a substance which kills or displaces mycotoxin-producing fungi, the levels of toxin contamination can be significantly reduced in the harvested material.

Saponins are a class of compounds, each consisting of a sapogenin portion and a sugar moiety. S. Budavari, ed., *The Merck Index,* 11th ed., Merck & Co., Inc., Rahway, N.J., 1990, p. 1328. Saponins for use in the present formulations are a type of sterol glycoside widely distributed in plants, where each saponin consists of a sapogenin

and at least one sugar moiety. The sapogenin comprises a steroid or a triterpene and the sugar moiety may comprise glucose, galactose, pentose, or methylpentose. The more preferred saponins for use in the present invention are derived yucca plants, with the most preferred being saponin extracts from *Yucca schidigera* or *Y. valida.* A variety of structurally related saponins are known, the most variable structural feature being the glycosylation pattern. *Id.* Saponins also can contain additional modifications, such as the sarasaponins which are saponins with a steroid attached, and saponin structure can be modified by any number of enzymatic, chemical and/or mechanical means known in the art. Saponins from *Yucca schidigera* contain steroidal saponins, with the major sapogenins being sarsapogenin and tigogenin. The sarsaponin yields on hydrolysis, sarsasapogenim (sarsasapogenim 5-beta, 20-betaF, 22-deltaF, 25-betaF; also known as spirostan-3-beta-01 and parigenin), glucose and galactose. The sarasapogenim has a molecular formula of $C_{27}H_{44}O_3$. Nobel, Park S., *Agaves,* Oxford Univ. Press, New York, 1994.

The saponins for use in the present invention can be isolated from a natural source, be wholly or partially synthetic, or be produced by recombinant techniques. As an example, they can be produced and/or isolated from various plant parts including fruit, leaf, seed and/or root, using means known in the art, from a variety of sources including the various plants known to produce them, ranging from yucca, quillaja, agave, tobacco, licorice, soybean, ginseng and asparagus to aloe woods. Saponins for use in the present invention are preferably non-toxic to humans and higher animals at the concentrations used. Most preferably the saponin for use in the present invention is non-toxic food grade, the source being yucca plants. Even more preferred are the saponins from *Yucca schidigera* or *Y. valida* and their equivalents. For both phytotoxicity control as well as toxicological safety, preferred saponins are from *Yucca* spp. and preferred saponins that do not bind cholesterol include those from asparagus. The saponins generally are prepared by a cold press extraction process and the resulting liquid extract analyzed by HPLC for saponin concentration. The yucca fiber also can be used; it is typically sundried, mulled and sized by screening.

Derivatives of these compounds which produce a formulation having the desired antipathogenic and/or phytotoxic effect are considered equivalents of the invention. Depending on its structure, a given saponin can have a particular property and find use with the present formulations. Generally, saponins can be used in a variety of formulations, for example, as insecticides, fungicides, biocides generally, surfactants, emulsifiers, flavorings and feed supplement formulations. These diverse activities are attributable to the chemical make up of a particular saponin and usually depend on the source form which the saponin is derived. For example, saponins derived from Japanese Camellia control the growth of mosquito larvae. Saponins from sources other than Yucca plants can be used as active agents in insecticidal compositions. Saponins from Yucca plants are used to remove cholesterol from foodstuffs and as a biocidal treatment and anti-caking/emulsification agent for waste sludge and other biomass systems. Sarasaponins derived from agave plants, for example, find use as wetting agents and with other compounds for the treatment of human fungal diseases.

Of particular interest are the use of saponins as synergists to increase the effect of at least one other compound present in a formulation for application against various pathogens, toxin producing microorganisms or their toxic metabolites whereby the combined action of the formulation ingredients is greater than the sum of their separate, individual actions and/or range of activity. The use of a synergist allows for a reduction in the concentration of one or more other ingredients in a given formulation while substantially maintaining efficacy of the formulation. Combination of such a component with other ingredients of the formulation can be accomplished in one or more steps at any suitable stage of mixing and/or application. It is preferable to select a saponin that optimally increases the antipathogenic and toxin reducing effect of the formulation. Generally an effective amount of saponin is of the range of about 0.1 to 3% and most preferably about 0.25% v/v aqueous solution of $10^0$ brix saponin extract. $10^0$ brix is a term of art in sugar chemistry. The brix degrees equals the percent by weight of sugar in the solution. Hawley, ed., *The Condensed Chemical Dictionary,* 10th ed., Van Nostrand Reinhold, New York, 1981, p. 149.

A saponin affording the desired activity is most preferably selected for combination with at least one of the various aldehydes, particularly aromatic aldehydes which can be used for direct killing of fungal or insect pathogens and/or for the induction of systemic plant resistance to various fungal or insect pathogens. Thus, these antipathogenic agents preferably comprise at least one saponin as a synergist in combination with at least one pathogen growth modulating aromatic aldehyde in an amount sufficient to control growth of a target pathogenic organism. By "controlling growth" is intended killing the pathogen and/or slowing or arresting its proliferation. Pathogens include insects, fungi and other microorganisms that negatively affect the plants that they colonize.

The subject formulations preferably include, in addition to saponin, the pathogen growth modulating compound as shown in formula (1) below.

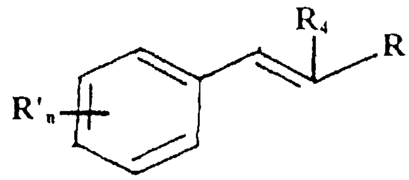

(1)

wherein R represents $-CH_2OH$ or -CHO; n is an integer from 0 to 3; and each R' independently represents OH or an organic substitutent containing from 1 to 10 carbon atoms and from 0 to 5 heteroatoms, wherein the total number of carbon and heteroatoms in all R' substitutents of said compound is no more than 15, and $R_4$ represents hydrogen or an organic constituent containing from 1 to 10 carbon atoms. These aromatic aldehyde compounds include natural products such as cinnamic aldehyde. Also of interest are alpha substituted aldehydes, such as α-hexyl cinnamic aldehyde (HCA), coniferyl aldehyde, and closely related compounds.

A number of the aromatic and aliphatic aldehydes which find use in the subject invention, such as benzaldehyde, acetaldehyde, cinnamaldehyde, piperonal, and vanillin are generally regarded as safe (GRAS) synthetic flavoring agents (21 CFR §172.515). HCA was in public use before the 1950's and today is widely used in consumer preparations (soaps, detergents, creams, lotions, perfumes) (Monographs on fragrances raw materials. Food Cosmet. Toxicol. 12: suppl., 915, 1974). HCA was granted GRAS (generally recognized as safe) status by FEMA (Flavoring Extract Manufacturers' Association. Survey of flavoring ingredient usage levels. No. 2569. Fd. Technol., Champaign, 19: (part 2) 155, 1965) in 1965 and is approved by the US FDA for use in food (21CFR121.1164). The Council of Europe (1970) (Council of Europe. Natural and Artificial Flavouring Substances. Partial Agreement in the Social and Public Health Field. Strasbourg, List A(1), Series 1, no. 129, p. 55, 1970) included HCA in the list of admissible artificial flavoring substances at a level of 1 ppm. Various of these compounds have been reported to have inhibitory activity against *C. botulinum* spore germination. Bowles and Miller, *G. Food Protection* (1993) 56: 788-794.

A preferred compound included in the subject formulations is shown in formula (2) below:

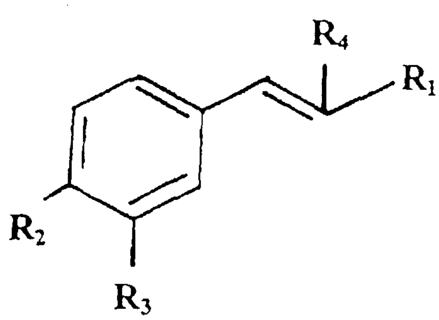

(2)

wherein $R_1$ represents-CHO, $R_2$ represents -H, -OH or an organic substituent containing from 1 to 10 carbon atoms, $R_3$ represents -H, a methoxy group or organic substituent containing from 1 to 10 carbon atoms, and $R_4$ represents a hydrogen or an organic substituent containing from 1 to 10 carbon atoms. Of particular interest are aromatic aldehydes. Examples of aromatic aldehydes of use in the present invention are cinnamic aldehyde ((3) below):

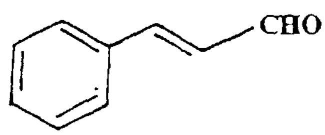

(3)

and coniferyl aldehyde ((4) below):

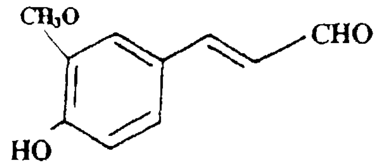

(4)

Other compounds of interest include analogs of the compound of formula (1) such as compounds substituted at the alpha position with an alkyl, such as a hexyl group, or a branched alkyl group such as an amyl group. Generally the group at the alpha position is from C-5 to C-10. Such compounds include alpha hexyl cinnamaldehyde and alpha amyl cinnamaldehyde. The chemical structure of alpha-hexylcinnamic aldehyde (HCA) is shown in (5) (below):

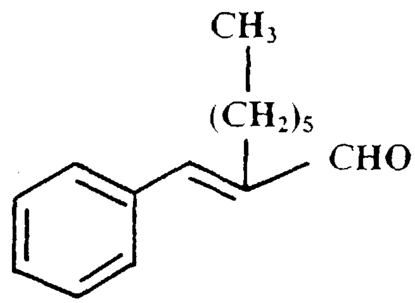

(5)

The Chemical Abstracts Service (CAS) name of HCA is 2-(phenylmethylene) octanal and the CAS Registry Number is [101-86-0]. The compound is also described by the chemical name of 2-hexyl-3-phenyl-2-propenal. The formula of the compound is $C_{15}H_{20}O$ and the molecular weight is 216.3. HCA is a low to moderately volatile compound, having a vapor pressure of $70 \times 10^{-5}$ mm Hg at 25°C. Its parent compound, cinnamic aldehyde, has a vapor pressure approximately 40 times higher ($2970 \times 10^{-5}$ mm Hg at 25°C).

The aromatic and aliphatic aldehydes of the subject invention can be prepared by various synthetic methods known to those skilled in the art. For example, *see. J* March, ed., Appendix *B. Advanced Organic Chemistry: Reactions, Mechanisms, and Structure,* 2nd Ed., McGraw-Hill, New York, 1977. Cinnamaldehyde can be prepared synthetically, for example, by oxidation of cinnamyl alcohol (Traynelis *et al., J. Am. Chem. Soc.* (1964) 86:298) or by condensation of styrene with formylmethylaniline (Brit. patent 504,125). The subject aldehydes also can be obtained by isolation from natural sources. For example, cinnamaldehyde can be isolated from woodrotting fungus, *Stereum subpileatum.* Birkinshaw *et al., Biochem. J.* (1957) 66:188.

HCA can be synthesized as described, for example, in USPN 5,055,621. On a laboratory scale, HCA can be synthesized by reaction of benzaldehyde with octanal under a nitrogen atmosphere (aldol condensation) (Personal Communication, Eric Walborsky, Firmenich Chemical Manufacturing Center, Port Newark, New Jersey). The reaction is conducted in a stirred flask charged with methanol, 309 ppm diphenylamine, potassium hydroxide and benzaldehyde. Following the slow addition of octanal, the reaction mixture is brought to a pH of 7.5-9.5 with acetic acid. Following evaporation of methanol and wash of the reaction mixture with water, the organic phase is transferred to a distillation unit. Approximately 20-24% of the pot charge is removed as benzaldehyde and "lights", with the remaining distillate constituting alpha-hexylcinnamic aldehyde "heart cut." The "heart cut" is subjected to an additional fractionation, in which 1-5% (by weight) of the material is removed in "light" fractions, depending upon odor evaluation. The final product is a light yellow oil having a specific gravity of 0.955-0.965 at 20°C, a refractive index of 1.548-1.562 at 20°C, a boiling point of 305°C at 1 atmosphere, and a melting point of 26°C.

HCA also can be obtained from Firmenich; their product is composed principally of the (E)-cis isomer (93.8% maximum), and the (Z)-trans isomer (6% maximum). Among minor components is the self aldol condensation product of octanal (1-1.5% (Personal Communication, June Burkhardt, Firmenich, Plainsboro, New Jersey). The commercial product is stabilized with the addition of 0.04% 2, 6-di-tert-butyl-*p*-cresol (butylated hydroxytoluene or BHT), which serves as an antioxidant (Technical Data Sheet, Hexylcinnamic aldehyde 907600, Revision 853, Firmenich Inc., Plainsboro, New Jersey). HCA can be isolated from rice where it has been reported to occur naturally. (Givaudan-Roure Index, Givaudan-Roure Corporation, Clifton, New Jersey, 1994, p. 89).

The subject saponin and aldehyde compound compositions can be used either alone or in combination with other active or inactive substances. In some instances, the efficacy of the formulation can be increased by adding one or more other components, *i.e.,* a compound other than saponin or a compound of formula (1), to the formulation. It is

preferable that the additional component(s) minimize phytotoxicity of a particular formulation while increasing the antipathogenic effect of the formulation. Of particular interest is the addition of adjuvants to a formulation. By "adjuvant" is intended a substance added to a formulation to aid the operation of the main ingredient. A spray adjuvant performs this function in the application of an agricultural chemical. An effective spray adjuvant may be formulated to contain one or more surfactants, solvents or co-solvents. Systems containing surfactants, water and oily components have many other possibilities of forming ordered phases; the surfactant can organize itself into aggregates of various shapes to create micelles, with a first order phase as one of the possibilities. The surfactant also can collect at the interface between interpenetrating oil and water phases to create a microemulsion. For example, the formulation can be rendered substantive by including an emulsifier such as Tween 80. Other detergents which can be used include anionic detergents such as those described in U.S. Patent Application No. 4,978,686. Generally, detergents and other agents used in the formulation do not detract from the pesticide properties of the aromatic aldehydes but do increase the substantive properties of the formulation *(see* for example, U.S. Patent Application No. 4,477,361) and may improve the pesticide properties.

Additional components such as an aqueous preparation of a salt of a polyprotic acid such as sodium bicarbonate, sodium sulfate, sodium phosphate or sodium biphosphate optionally can be included in the formulation, to increase the antifungal or insecticidal properties of the formulation. The resulting emulsion is diluted to an appropriate concentration for use.

The most effective formulations for compositions that include a saponin and compounds of formula (1), (2), (3), (4) and/or (5) can be determined using protocols such as those described in the Examples and other methods known to those of skill in the art. Optimization of each formulation can be achieved as desired by including at least one compound other than saponin to increase the effect of at least one other compound present in the formulation. Each formulation is evaluated for its effect on specific pests and/or plant host using saponin and any of the compounds of formula (1) as well as other components of the formulation such as Tween 80 and/or sodium bicarbonate, with the combination and effective amount of each component adapted for a particular application to minimize phytotoxicity while maintaining or increasing the antipathogenic effect of the formulation.

Of particular interest is optimization of the formulation to increase the mean disease resistance against a broad range of pathogens. The effective amount of each component can be determined by systematically varying the amount of that component in a test formulation, treating a plant of interest with the test formulation, and monitoring the level of pest infestation as compared to an untreated control plant. As an example, various concentrations of saponin can applied as a separate spray to a plant in applications which employ a series of formulations containing variable amounts of a given aromatic aldehyde and/or other component. An effective amount of a test component may be identified as the amount that controls the growth of the pathogen on the plant host, thus reducing the disease rating of the plant. The mean disease resistance (MPDC) can be calculated for each particular application. MPDC is defined by the formula:

$$\text{MPDC} = \frac{(\text{MDIC-MDIT})}{\text{MDIC}} \times 100$$

and

MDIC = Mean % of disease incidence in untreated controls
MDIT = Mean % of disease incidence in the treatment

Generally, for effective pathogen control the mean percentage of disease control (MPDC) is greater than 60%, preferably at least about 70%. An effective amount of a saponin is typically measured as about an equivalent in activity of a saponin extract control from *Y. schidigera* comprising about 0.05% to 3% v/v and preferably about 0.25% v/v aqueous solution of 10° brix saponin extract.

The formulations also are evaluated for phytotoxicity; it therefore is important that at least one evaluation of the toxicity of the formulations be on living plants of the host variety. Phytotoxicity can be rated as follows in order of increasing severity of toxicity: 0-plants without any symptoms; 1-very slightly browning of hypocotyl (no other symptoms); 2-some wilting of plant, dying of lower leaves, some browning of vascular system; 3-wilting of entire plant, leaves dying, hypocotyl with external and internal symptoms; 4-necrosis of stem, plant dying. It is preferable that the formulation used have a phytotoxicity rating of 2 or less, more preferably 1 or less. As an example, the effects of cinnamic aldehyde in a range from 0.1 ppm to 25,000 ppm on powdery mildew is evaluated. The mean disease control can be increased by using higher doses of cinnamic aldehyde, and/or adding other compounds of formula (1), or by increasing the substantiveness of the formulation by adding detergent, and the like.

An effective growth modulating amount of a test component may be identified as the amount that decreases the extent to which a pest colonizes a host plant, either by killing the pest or preventing its reproduction. An effective

growth modulating amount of one or more compounds of formula (1), (2), (3), (4), or (5) is generally about 0.01 g/l to 25 g/l, more preferably about 1 g/l to 20 g/l, and most preferably about 5 g/l to 10 g/l. In a preferred embodiment, the formulation includes saponin and an effective growth modulating amount of α-hexyl cinnamic aldehyde, cinnamic aldehyde and/or coniferyl aldehyde in a formulation containing Tween 80 as an emulsifier and optionally sodium bicarbonate. The preferred formulation is an emulsion which contains saponin in addition to α-hexyl cinnamic aldehyde, cinnamic aldehyde and/or coniferyl aldehyde (0.5% to 10% by weight) and may include the salt of an aprotic acid (8% to 12% by weight) and the balance water. Formulations with 6-12% of an aprotic acid are preferred. Generally, the total amount of aldehyde(s) present in the formulation is 5 % or less. The preferred formulation for treating powdery mildew, rust and spores, as well as aphids is an emulsion which contains α-hexyl cinnamic aldehyde, cinnamic aldehyde and/or coniferyl aldehyde (0.001% to 10% by weight), the salt of a polyprotic acid (4% to 12% by weight), an emulsifier (1% to 4% by weight), and the balance water. The formulations are effective without the use of antioxidants other than the inherent antioxidant properties of particular aldehydes, for example, coniferyl aldehyde.

Other compounds can be used alone or in combination with the compositions, for example $H_2O_2$, which is known to kill particular fungi such as *A. flavus* in alkaline environments. An antifreezing component such as glycerol, propylene glycol, ethylene glycol and/or isopropyl alcohol, and a gum or gum-like material as xanthan gum, acacia gum, gelatin, hydroxypropyl methyl cellulose and the like can also be included such as those described in U.S. Patent No. 5,290,557 so long as the amount used does not injure the plant and/or any harvest such as fruit. Additionally, for use preharvest, compounds which induce non-systemic or systemic plant resistance to various fungi may be used to control colonization and/or growth of certain fungi under field conditions.

Stability of the formulation can be evaluated by a variety of methods, including accelerated tests in which a formulation of interest is exposed to elevated temperatures over a set time. Samples of the formulations are taken at regular intervals and analyzed chemically by methods known to those skilled in the art to determine the rate and nature of degradation. For example, HCA can be analyzed by Gas Liquid Chromatography (GLC), using a 30 meter non-polar polydimethylsiloxane capillary column (e.g. HP-1, Hewlett-Packard, or SPB-1, Supelco) and a flame-ionization detector. Using helium as a carrier gas (8 ml/min.) and a column temperature of approximately 240°C, the (E)-cis isomer (major component) has a retention time of approximately 6.0 minutes and the (Z)-trans isomer (minor component) has a retention time of approximately 6,3 minutes.

For applications where the formulation is to be used to prepare the ground or other growth substrate for planting of host plants susceptible to particular pathogens, to apply to an already infested growth substrate, or to harvested material the formulations of the subject invention can be added directly to the rhizosphere, the substrate or the harvested material, or they can be bound to a solid support or encapsulated in a time release material. Where a solid carrier is used, materials which can lead to oxidation of the active aldehydes should be avoided. Examples of delivery systems include starch-dextran, and the like. *See* Yuan *et al., Fundamental and Applied Toxicology* (1993) 20: 83-87, for examples of delivery systems. Also *see* Kawada *et al.* (1994) 10: 385-389.

In addition to the saponin and the specific compounds of the formulas (1), (2), (3), (4) and (5) as set forth above, derivatives of any of these compounds that produce a compound of the formula identified above upon action of a biological system on the derivative are considered to be equivalent to compounds of the invention. Thus application of precursor compounds to plant parts or tissues or harvested materials would be equivalent to the practice of the present invention. Biological conversion of precursor compounds into aromatic aldehydes is described in, for example, U.S. Patent Application No. 5,149,715 and references cited therein. *See* also Casey and Dobb *Enzyme Microb. Techol.* (1992) 14: 739-747. Examples of precursor compounds include those in pathways relating to acquired and/or systemic resistance to plant resistance to plant pathogens such as those in the amino acid ammonia lyase pathways, and include phenylalanine (to produce cinnamic acid). Other precursor compounds of interest include those in the biological pathways for the production of lignins and coumarins, for example tyrosine to produce p-coumaric acid. Accordingly, precursors and derivatives of these compounds which produce a formulation having the desired antipathogenic and toxin reducing effect are considered equivalents of the invention.

The method of the present invention is carried out by introducing into a target pathogenic organism a sufficient amount of an antipathogenic agent to impair growth and/or viability of the target pathogenic organism. A formulation containing the antipathogenic agent is introduced to a plant tissue or part either pre- or post-harvest. Methods of application include spraying, pouring, dipping, injecting, and the like, the active ingredient in the form of a concentrated liquid, solution, suspension, powder and the like. For example, the formulation is sprayed on as a wet or dry formulation to the surface and/or underside of the leaves or other plant tissue or part of a plant infected with a plant pathogen, or of a plant susceptible to infestation with a plant pathogen, preferably to the point of run off when a wet formulation is used. The plants can be sprayed prior to or after infestation, preferably prior to infestation. However, in order to minimize damage to the host plant, where feasible, it is preferable to treat older plants, as young green leaves tend to be more sensitive to phytotoxicity.- Alternately, the formulation can be applied wet or dry, either as part of an irrigation schedule or as a separate application, to the rhizosphere where it can contact the roots and associated pathogenic organisms which colonize the roots. In some instances, time-release formulations may find use, particularly for applications to the

rhizosphere, or to post-harvest materials. The amount of the formulation that is applied either to the plant itself or to the rhizosphere will depend upon the degree of infestation and to some extent upon the formulation and the specific compound(s) used in the formulation and therefore is empirically determined for best results.

The method of introducing the active ingredient(s) of the formulation into the target organism can be by direct ingestion by the pest organism from a treated plant surface, or by feeding of a pest organism on a nutrient-providing surface of a host entity, which is colonized by the target pest organism, which host either contains or has on its surface the antipathogenic agent. The presence of the anti-pathogenic agent on a nutrient-providing surface of a host plant can be a result of direct contact of the antipathogenic agent with the plant part or it can be by elaboration from the host plant as a result of induction of systemic resistance as a secondary effect to prior treatment of the plant with the antipathogenic agent, or as a result of genetic modification of the host plant.

Depending upon the target organism, the saponin and/or aldehyde to be used can be microencapsulated or coupled to a solid support, optionally through a linker such as a binding domain derived from a polysaccharidase, where the solid support is a polysaccharide such as cellulose, particularly microcrystalline cellulose. The preparation of cellulose binding domains is described in U.S. Patent Nos. 5,340,731; 5,202,247 and 5,166,317. Binding domains from scaffold proteins also can be used. *See* Shoseyev *et al.* (PCT application PCT/US94/04132 WO-A-9424158). The saponin and/or aldehydes can be coupled to the binding domains or other solid support, with or without a cleavable bond, using methods well known to those skilled in the art. Solid or microencapsulated forms of the active ingredients are particularly useful for treating or preventing infestations of soil-borne pathogens. Analytical chemical techniques are used to determine and optimize rate of release of the active ingredient from the solid or microencapsulated form. For qualitative purposes, gas chromatography techniques can be used to determine the amount of aldehyde released. For example, samples of encapsulated (pelletized) product are mixed with the soil types selected and sampled at different time periods to measure release. Volatile gases released from the formulation can also be analyzed. The activity and stability of foliar and drip irrigation applications of the formulations over time can also be evaluated using the GC methodology using methods known to those skilled in the art. Methanol or alcohol extractions of the formulations also can be prepared for HPLC analysis.

One or more components of the present formulations can be produced in the plant of interest by modulating the expression of one or more genes encoding one or more enzymes or an enzyme pathway or cluster required to control the level of the compound of interest in the plant, plant part, plant cell, specific plant tissue and/or associated with a particular stage of plant growth. The enzyme can be in a biosynthetic pathway or a degradation pathway and the regulation will be up or down, respectively, to modulate expression of either an endogenous plant gene or a transgene supplied exogenously to the plant. An indigenous plant gene is one which is native to the genome of the host plant. An endogenous plant gene is one that is present in the wild-type genome of the plant host of interest. It may be an indigenous gene or a gene that is present as a result of infection of the plant (for example, a viral gene) or otherwise naturally incorporated into the plant genome. The host plant also can be modified by recombinant means or by traditional plant breeding methods to introduce one or more genes exogenous to the host plant which encode enzymes that control the level of the compound of interest and as such are in the synthetic pathway for saponin or one or more compounds of formula (1), (2), (3), (4) or (5). By modulation of gene expression is intended control of production of a gene product of interest at the level of transcription, translation and/or post translation. The level of the compound of interest is controlled by modulating the expression of one or more endogenous genes or transgenes encoding one or more enzymes required to synthesize the compound of interest.

Methods for modulating gene expression in plants are known in the art. Variation in growth conditions or exogenous application of compounds to a plant can affect gene expression. For example, the formulations of the present invention can be used to induce systemic plant resistance through modulation of endogenous gene expression. At the molecular level, gene expression depends substantially on the transcription, translation and termination control regions which regulate expression of a structural gene coding region. By exploiting the plant signals which regulate these control regions or by the direct recombinant manipulation of the control regions, expression of a gene encoding an enzyme required to control the level of saponin, for example, can be modulated. Where the transgene is exogenous to a plant host, the transgene will include control regions that are selected and designed to achieve the desired level and timing of gene expression. As appropriate, the control regions are homologous (native) or non-homologous (non-native) to the gene of interest. By "homologous" is meant that the control region(s) is from or substantially similar to a control region normally associated with the gene of interest. By "non-homologous" is meant that the control region(s) originates from a different nucleotide source or sequence or is substantially different from the control region(s) normally associated with the gene of interest. For example, if the enzyme coding sequence is non-homologous in source as compared to the control regions, in order to have expression of the gene in a plant cell of interest, transcriptional and translational initiation regulatory regions or promoters functional in these plant cells are provided operably linked to the coding sequence. Transcription and translation initiation signals functional in plant cells include those from genes which are indigenous or endogenous to a plant host or other plant species, and direct constitutive or selective expression in a plant host, and include sequences from viruses such as CaMV which infect plants.

Of particular interest are the gene control regions that selectively regulate structural gene expression in a plant, plant part, plant cell, specific plant tissue and/or associated with a particular stage of plant growth. Preferred are those control regions that are known in the art, and in particular, transcriptional control regions or promoters that can be used to modulate the expression of a gene encoding an enzyme required to control the level of saponin and/or a component of formula (1), (2), (3), (4), (5) in a plant, plant part, plant cell, or specific plant tissue and/or are associated with a particular stage of plant growth. For example, promoters providing for differential expression patterns in fruit are described in USPN 4,943,674 and USPN 5,175,095; seed in USPN 5,315,001; and in rapidly developing tissues and tender shoots in USPN 5,177,011.

A preferred method for producing a desired component of the present formulations in a plant host is through recombinant DNA means, particularly by modifying the level of saponin and/or at least one compound of the formula (1), (2), (3), (4), (5) in plant tissues of interest through construction of transgenic plants using recombinant techniques known in the art. The methods involve transforming a plant cell of interest with an expression cassette functional in a plant cell comprising as operably linked components in the 5' to 3' direction of transcription, a transcriptional and translational initiation regulatory region, joined in reading frame 5' to a DNA sequence encoding one or more enzymes capable of modulating the production and/or required to produce the compound of interest, and translational and transcriptional termination regions. Expression of an enzyme required to produce the compound of interest provides for an increase in production of the compound as a result of altered concentrations of the enzymes involved in the compounds' biosynthesis. Of particular interest is the selective control of saponin, cinnamic and/or coniferyl aldehyde production in plant tissues such as leaves, roots, fruits and seeds.

For selective control of saponin biosynthesis in a tissue of interest, plant cells are transformed with an expression cassette comprising DNA encoding a structural gene for one or more enzymes required to synthesize saponin and capable of increasing the amount of this compound in the tissue of interest. Similarly, for cinnamic aldehyde biosynthesis in a tissue of interest, plant cells are transformed with an expression cassette comprising DNA encoding a structural gene for one or more enzymes required to synthesize cinnamic aldehyde and capable of increasing the amount of cinnamic aldehyde in the tissue of interest. Of particular interest are those genes encoding one or more enzymes capable of metabolizing a precursor compound required for the biosynthesis of the saponin, cinnamic and/or coniferyl aldehyde compound of interest from substrates normally found in a plant cell. More particularly of interest is the transgenic expression of at least one compound of the formula (1), (2), (3), (4), (5) and a saponin.

DNA constructs for expressing a gene of interest are prepared which provide for integration of the expression cassette into the genome of a plant host. Integration can be accomplished using transformation systems known in the art such as *Agrobacterium,* electroporation or high-velocity microparticle-mediated transformation. Depending upon the application, saponin or one of the other compounds of interest can be preferentially expressed in a tissue of interest and/or a particular organelle. Tissue specificity is accomplished by the use of transcriptional regulatory regions having the desired expression profile. Translocation of the enzyme to a particular organelle is accomplished by the use of an appropriate translocation peptide. Methods for tissue and organelle specific expression of DNA constructs have been described and are known in the art.

To verify regulation and expression of the gene of interest, various techniques exist for determining whether the desired DNA sequences present in the plant cell are integrated into the genome and are being transcribed. Techniques such as the Northern blot can be employed for detecting messenger RNA which codes for the desired enzyme. Expression can further be detected by assaying for enzyme activity or immunoassay for the protein product. Most preferably the level of the compound of interest present in a plant host is measured using methods known in the art. A desired phenotype, for example, is increased saponin and/or aromatic aldehyde content in a plant tissue of interest as measured by expression of the gene of interest and/or the level of saponin present in the plant host as compared to a control plant.

For introduction of one or more compounds of the present formulations to the target organism, a plant host expressing a gene encoding an enzyme required to control the level of the compound of interest results in the exposure of a target organism to at least one component of the antipathogenic formulation. In another embodiment, selective expression of the gene of interest induces systemic plant host resistance to pathogen attack or colonization. At least one component of the antipathogenic formulation can be expressed by the plant host and optionally other components of the antipathogenic formulation are exogenously applied to the plant host so that the combination elicits the desired antipathogenic effect when either directly or indirectly introduced to the target organism. Transgenic plants having an increased ability to accumulate saponins, in addition to autoprotection against plant pathogens can be used as a source of saponins for extraction and subsequent use as a chemical pesticide.

Accumulation of aromatic aldehydes can be achieved by downregulating the expression of specific plant genes that encode enzymes which either cause further metabolism of the desired aldehydes or divert metabolic intermediates away from the desired aldehydes. In the case of cinnamaldehyde, for example, this involves downregulating the expression of cinnamate 4-hydroxylase (CA4H) and cinnamic alcohol dehydrogenase (CAD). CA4H ordinarily diverts some cinnamic acid away from cinnamaldehyde to produce *p*-coumaric acid, itself a metabolic intermediate.

Reducing CA4H activity alone generally is not sufficient to cause accumulation of cinnamaldehyde because CAD can rapidly convert cinnamaldehyde to cinnamyl alcohol, which then becomes incorporated into lignin or accumulates as glycosides. Simultaneously reducing both CA4H and CAD activities results in increased metabolic flux from cinnamic acid into cinnamaldehyde and decreased conversion of cinnamaldehyde into cinnamyl alcohol. Some cinnamaldehyde becomes incorporated into lignin but cinnamaldehyde (either free or as glycosides) also accumulates to above-normal levels, particularly at times when the biosynthesis of cinnamic acid is elevated. This occurs when the level of phenylalanine ammonia lyase (PAL; the first and rate-limiting step in general phenylpropanoid metabolism, Hahlbrock and Scheel, (1989) *Annu. Rev. Plant Physiol. Plant Mol. Biol.* 40:347-369) activity is high, a situation that naturally occurs in plants in response to a wide range of stimuli including invasion by fungal pathogens and mechanical damage associated with wounding and insect feeding.

A number of plant CA4H and CAD genes have been cloned and their sequences are available from GenBank. Portions of these genes that include nucleotide sequences that are conserved among different plant species can be used directly in a plant expression vector (antisense or sense orientation) to suppress the expression of the corresponding endogenous genes (*e.g.,* Pear *et al.* (1993) *Antisense Res. and Develop. 3:181-190,* Napoli *et al.* (1990) *The Plant Cell* 2:279-289. *See* also USPN 5,107,065). More preferably, these conserved gene sequences are used to isolate CA4H and CAD cDNA clones from a cDNA library of the plant species that is to be modified. The resulting cDNA clones, or portions thereof, are then introduced into a plant expression vector (antisense or sense) and used to transform the plant(s) of interest. DNA constructs according to the invention preferably comprise a sequence of at least 50 bases which is homologous to the endogenous CA4H or CAD genes.

A recombinant DNA molecule is produced by operatively linking a vector to a useful DNA segment to form a plasmid that can be used for plant transformation. A vector capable of directing the expression of RNA from a cloned portion of a gene is referred to herein as an "expression vector." Such expression vectors contain expression control elements including a promoter. Typical vectors useful for expression of genes in higher plants are well known in the art and include vectors derived from the Ti plasmid *of Agrobacterium tumefaciens* described by Rogers *et al., Methods in Enzymology* (1987) 153:253-277. A common promoter that is used to provide strong constitutive expression of an introduced gene is the cauliflower mosaic virus (CaMV) 35S promoter (available from Pharmacia, Piscataway, NJ). Either constitutive promoters (such as CaMV 35S) or inducible or developmentally regulated promoters (such as the promoter from a PAL gene or the endogenous CA4H or CAD genes) can be used. Use of a constitutive promoter tends to affect functions in all parts of the plant, while use of an inducible or developmentally regulated promoter has the advantage that the antisense or sense RNA is produced substantially only in a desired tissue and under the conditions it is required. The use of developmentally regulated promoters is preferred because the down-regulation of phenylpropanoid biosynthesis is known to be capable of producing undesirable side-effects on the development of transgenic plants containing a heterologous PAL gene (Elkind *et al.* (1990) *Proc. Nat. Acad. Sci.* 87:9057-9061.

A number of different transformation methods are available for the routine transformation of a wide range of plant species. One method that is particularly efficient for the transfer of DNA into dicotyledonous plants involves the use of *Agrobacterium.* In this method the gene of interest is inserted between the borders of the T-DNA region that have been spliced into a small recombinant plasmid with a selectable marker gene (for example encoding neomycin phosphotransferase II or phosphinothricin acetyltransferase). The recombinant plasmid is then introduced into an *Agrobacterium* host by transformation or triparental mating. The *Agrobacterium* strain carrying the gene(s) of interest is then used to transform plant tissue by co-culturing the bacteria with an appropriate plant tissue (*e.g.,* leaf disc) Transformed cells are selected in tissue culture using the appropriate selection agent and plants are then regenerated *(see* Horsch *et al.* (1985) *Science* 227:1229-1231. Other methods that have been used in the transformation of plant cells, and in particular the more recalcitrant crop plants, include biolistics and electroporation (for detailed protocols. *see Sanford et al.* (1993) *Methods in Enzymology* 217:483-509; and Potter (1993) *Methods in Enzymology* 217:461-478.

Once transgenic plants have been produced, conventional enzyme assays for CA4H and CAD are used to determine the level of suppression of enzyme activity achieved in different transformants. It is likely that only a small fraction of the transformants produced will have a sufficiently low residual enzyme activity to cause the accumulation of aromatic aldehydes without also producing some undesirable side effects on plant development. For this reason, a preferred method of producing the desired transformants with both CA4H and CAD suppressed is to introduce the two genes separately into different transformants and then combine them by standard sexual crosses. This permits a larger number of combinations of level of gene suppression to he evaluated at the same time.

An alternative to overproducing saponin or aromatic aldehydes in transgenic plants is to use the plant genes to confer on a microbial host the capability of synthesizing specific aromatic aldehydes and/or saponins. The resulting microbes may be used either to produce the aromatic aldehydes in a fermentation system or as a natural delivery system of the aromatic aldehydes in viable or non-viable microbial preparations. Yeasts, especially *Saccharomyces cerevisiae,* are preferred organisms for this purpose because they have already been engineered for high-level expression of PAL (Faulkener *et al. (1994) Gene* 143:13020) and a plant cinnamate 4-hydroxylase has been shown to function in yeast (Urban *et al.* (1994) *Eur. J. Biochem.* 222:843-850.

The expression of PAL introduces the capability to produce cinammic acid from phenylalanine, Two additional enzymic steps are required to produce cinnamaldehyde from phenylalanine. In plants, these steps are catalyzed by the enzymes cinnamate:CoA ligase (CL) and cinnamoylCoA reductase (CCoAR) but as 4-coumarateCoA ligase (4CL) can also use cinnamic acid as substrate (Knobloch, and Hahlbrock (1977) *Arch. Biochem. Biophys.* 184:237-248), 4CL can be used instead of CL. More than 20 cloned PAL genes and more than 6 4CL genes have been described in sufficient detail (GenBank) to facilitate their use in practicing the current invention. A gene for a CCoAR is obtained by applying standard gene cloning techniques to isolate a cDNA clone using as a probe sequence derived from the amino acid sequence of the N-terminus, or peptide fragments, of the purified protein. CCoAR has been purified and partially characterized from soybean cultures (Wengenmayer *et al.* (1976) *Eur. J. Biochem.,* 65:529-536; Luderitz and Grisebach (1981) *Eur. J. Biochem,* 119:115-124), spruce carnbial sap (Luderitz and Grisebach, *supra*), poplar xylem (Sarni *et al.* (1984) *Eur. J. Biochem.* 139:259-265) and differentiating xylem of *Eucalyptus gunnii* (Goffner *et al.* (1994) *Plant Physiol.* 106:625-632). The preferred method of purification is that of Goffner *et al.* (*supra*) because it results in a single protein band on SDS-polyacrylamide gels that an be used for protein sequencing. For the expression of α-hexyl cinnamic aldehyde, the gene that codes for the enzyme that catalyzes the addition of the α-hexyl group to cinnamic aldehyde also is inserted into the microbial host or plant. The gene can be cloned, for example, from rice plants.

The cloned genes are introduced into standard expression vectors and used to transform a microbial host, preferably yeast, by standard transformation techniques such as electroporation (Becker and Guarante (1991) *Methods in Enzymol.* 194:182-187) Standard enzyme assays are used to confirm the functional expression of the engineered genes and assays for aromatic aldehydes are used to select strains with maximal production. Because aromatic aldehydes have antimicrobial properties it is preferred to use expression vectors that will cause expression of the introduced genes only late in the growth cycle or in response to a chemical inducer. It may also be desirable to grow the engineered microbial host in an immobilized whole cell reactor (*e.g.,* Evans *et al.* (1987) *Biotechnology and Bioengineering* 30: 1067-1072) to prevent the aldehydes from accumulating in the culture medium.

The subject formulations and methods are useful for treatment of plants that are colonized by pathogenic organisms. These include flowering plants, grasses, including ornamental turf grass, bent grass, vegetables, cereals and fruits including tomato, potato, artichoke, strawberries, corn, cereal grains, onion, cucumber, lettuce, tobacco, and citrus such as orange, lemons, limes and grapefruit, as well as bell peppers and grapes, and fruit trees such as peach, apple and cherry, ornamentals such as roses and trees, particularly conifers. Also included are crops intended for consumption by fish, fowl and animals, including humans, directly or indirectly. By "directly or indirectly" is intended that the crops could be ingested, for example, by humans (direct consumption), or that it is the nonhuman animal or fowl which ingests the crop and is in turn ingested by humans (indirect consumption). Crops intended for consumption include tobacco, fish, animal and fowl fodder, crops intended for processing into alcohol or food products such as corn syrup, and the like.

Target pathogenic organisms include fungi that colonize a plant or plant part, particularly a surface of a plant part. The optimal time and method for applying the formulations is determined by the particular part of the plant colonized by a fungus and the time in the plant life cycle at which a particular infestation occurs or is likely to occur. For example, to treat powdery mildew, rust and other pathogens which colonize the leaves of the host plant, the host plants are sprayed to run off with a formulation of the invention. The amount of compound(s) of formula (1) used will vary depending in part upon the target pathogen and the host plant and can be determined empirically by evaluating the sensitivity of the target organism to the formulation and the phytotoxic effects of that formulation or the host plant. The plants can be sprayed prior to or after infestation, preferably prior to infestation. However, in order to minimize damage to the host plant, where feasible, it is preferable to treat older plants, as young green leaves tend to be more sensitive to phytotoxicity. Alternatively, transgenic crops can be used, which express one or more components of the formulation in an amount sufficient to inhibit growth of the pathogen and/or kill the pathogen. Preferably the component(s) is expressed in the tissue colonized by the pathogen, for example the leaves.

Of particular interest is treatment of plants affected by powdery mildew which is caused by target organisms the fungal family *Erysiphaceae.* For example, the following species cause powdery mildew in the indicated plants: *Erysiphe cichoracearum:* begonia, chrysanthemum, cosmos, cucurbits, dahlia, flax, lettuce and *zinnia; E. graminis:* cereals and grasses; *E. polgoni:* beans, soybeans, clovers, and other legumes, beets, cabbage and other crucifers, cucumber and cantaloupe, delphinium and hydrangea; *Microsphaera alni*: blueberry, catalpa, elm, lilac, oak, rhododendron, and sweet pea; *Phyllactinia sp.*: catalpa, elm, maple and oak; *Podosphaera leucotricha:* apple, pear and quince; *P*. *oxyacanthae:* apricot, cherry, peach and plum; *Spaelrotheca macularis:* strawberries; *S*. *mors-uvae:* gooseberry and currant; *S*. *pannosa*: peach and rose; and *Uncinula necator*: grape, horse chestnut and linden.

Also of particular interest is the treatment of plants affected by rust caused by *Basidiomycetes,* particularly of the order *Uredinales.* There are about 4,000 species of rust fungi. The most important rust fungi and affected plants include: *Puccinia:* rust of numerous hosts such as wheat and all other small grains (*P. graminis*); yellow or stripe rust: wheat, barley and rye (*P. striiformis*); leaf or brown rust: wheat and rye (*P. recondita*); leaf or brown dwarf rust of barley (*P. holdei*) crown rust of oats *(P. coronata*); corn rust (*P. sorghi*) southern or tropical corn rust *(P. polysora*) sorghum

rust (*P. purpurea*); and sugarcane rusts (*P. sacchari* and *P. kuehnii*).

*Puccinia* also causes severe rust diseases on field crops such as cotton (P. stakmanii), vegetables such as asparagus (*P. asparagi,* and flowers such as chrysanthemum (*P. chrysanthemi*)*,* hollyhock (*P. malvacearum*), and snapdragon (*P. antirrhini*)*. Gymnosporangium,* causes the important cedar-apple rust (*G. juniperi-virginianae)* and hawthorn-cedar rust (*G. globosum). Hemileia,* causes the devastating coffee leaf rust. (*H. vastatrix). Phragmidium,* causes rust on roses and yellow rust on raspberry.

Other fungal species that cause rusts and are treatable using the subject formulations include *Uromyces:* legumes (bean, broad bean, and pea) (several *Uromyces* species) and carnation (*U. caryophyllinus*); *Cronanium:* severe rusts of pines, oaks, and other hosts, such as the white pine blister rust (*C. ribicola*); fusiform rust of pines and oaks (*C. quercuum* f. sp. *fusiforme*); eastern gall or pine-oak rust (*C. quercuum* f. sp. *virginianae*); pine-sweet fern blister rust (C. *comptoniae*); pine-Comandra rust (*C. comandrae*); and southern cone rust (C. *strobilinum*). Others include Melampsora, which causes rust of flax (*M. lini); Coleosporium,* which causes blister rust of pine needles (*C. asterinum*); *Gymnoconia,* which causes orange rust of blackberry and raspberry; Phakopsora, which causes the potentially catastrophic soybean rust (*P. pahyrhizi*); and *Tranzschelia,* which causes rust of peach.

The subject formulations and methods are also useful to prevent and treat infestation by organisms that colonize other parts of plants, such as the roots and fruit. For fungi that affect plant roots, such as *Fusarium sp., Aspergillus,* and *Verticulum* (including *V. alboatrium* and *V. dahlise*), infestations are preferably treated by foliar application of the subject formulations, with the active ingredient reaching the roots by translocation or by induction of a systemic acquired resistance (SAR). To treat fruit infestations, such as black spot, the subject formulations are applied during and after fruit development, preferably directly to the developing fruit. Other target fungal organisms include *Phragmidium spt; Diplocaopan rosae; Sphaerotheca tannosa; Oibiapsis sicula; Phytophoya taraesitica; Puccinia spp; Alternaria sp; Susaiun spp; Botrytis cinera; Sclerotinia Homoeocarca;* Dutch Elm disease (*Ceratocystis ulmi*) and oak wilt (*C. fagacearum*). Also included are blue-green algae (Cyanobacteria). Of particular interest is treatment of fungi that produce fumonisins, fusaric acid and/or their structural analogs.

The subject formulations are also useful against insects, particularly those of the orders *Orthoptera; Thysanoptera* which includes thrips; and *Homoptera* which include aphids, leafhoppers, white flies, mealy bugs, cicadas and scale insects. It is a theory of the invention that the insects which are susceptible to treatment with the subject formulations are those which harbor symbiotic bacteria in their gut. Accordingly, insects other than those listed which harbor symbiotic material also can be controlled with the subject formulations. Other target organisms include arachnids, particularly spider mites *(Anhropoda).* Additional insect targets include those of the orders *Coleoptera* such as corn root worm and cotton boll weevil; and *Lepodoptera,* which includes codling moth.

Of particular interest is treatment of phylloxera infestation in grapes. The root form of grape phylloxera *(Daktulosphaira vitifoliae* Fitch) is a devastating grape insect. Plant damage is not due solely to phylloxera feeding but rather is substantially due to invasion of secondary fungi, organisms which are not controlled by insecticides. Typically, phylloxera are found as deep as the roots of the host plant, which may be eight feet or deeper. Thus, for treatment of phylloxera infestation in grapes, one can deliver the formulation to the plant roots directly, such as by injection of liquid or solid formulation into the soil surrounding the roots. Alternatively, the formulations can be applied to the foliage or other accessible part of the plant and translocated to the root area through the plant vascular system or by induction of an SAR. As in the case of treatment of powdery mildew and rust, transgenic crops can be used for treatment of phylloxera; the preferred tissue of expression of components of formula (1) is the root.

Compounds of formula (1) can be used for treatment of phylloxera at different stages of growth. The compounds can be formulated or differentially applied to target particular stages of phylloxera growth such as the egg, nymph and adult stages. Efficacy of a particular formulation and treatment protocol can be evaluated by any number of means, such as assessing phylloxera mortality, feeding, and/or vacated feeding sites after treatment under field and/or laboratory conditions, or by improved health of the infested vines. In addition, efficacy of a particular formulation and treatment protocol can be evaluated through analysis of vine physiology in metabolizing, translocating and/or reacting to the formulation treatment by methods known in the art. Factors other than the normally tested dosage, formulation and timing of treatments also are considered to determine involvment of phylloxera phenology, plant phenology, plant health, presence of a graft union and cultivar (both of the scion and rootstock).

The subject methods and formulations are also useful for treating infestations of turfgrasses. Certain biotic agents of noninfectious diseases damage turfgrasses by competition. Algae frequently damage turfgrass by taking over and occupying the void that remains after turf is thinned by infectious disease. Blue-green grass algae (species of *Ajanobacteria*) develop as a black scum on the surface of overly wet soils. The algae reduce exchange of gases between air and soil and may also introduce chlorosis in plants. Black-layer is a physical condition that is primarily associated with putting greens on golf courses. It is noted especially in turfs with a high sand content. In addition, turfgrass fungal diseases can be treated using the subject formulations. *Sclerotinia* dollarspot, *Pythium* blight and *Rhizoctonia* blight are devastating diseases of various turfgrass species, especially bent grasses. The efficacy of the formulations for treatment of turgrasses can be determined by varying the components of the formulation, the application regime, volume of application and/or the rate of application. The evaluation can be conducted under field and/or laboratory conditions, with consideration given to environmental conditions such as humidity, temperature, light quality, quantity and intensity, soil type and fertility, moisture content, drainage and the like. The effect of formulation persistence and activity can be determined also by comparison of acropetal translocation and persistence with daily mowing and removal of the grass blades. The formulations may be applied at variable application rates and concentrations, and application regimes to obtain a desired level of disease control and phytotoxicity. Formulations containing saponin and compositions of formula (2) and (5) are preferred for this application, with formulae (3), (4) and (5) being more preferred.

Of particular interest is use of the subject formulations to control *Sclerotinia* dollar spot (*Sclerotinia homeocarpa*). *Sclerotinia* dollar spot is a widespread, persistent and costly disease that occurs on most turfgrass species throughout the world. Environmental conditions that favor dollar spot include long periods with high humidity and excess moisture. Low nitrogen fertility coupled with these environmental conditions increase the incidence of dollar spot disease. Fine-leafed grasses that form a dense and compact growth habit are especially at risk, making dollar spot a major threat to intensively managed golf course greens. Also of particular interest is use of the formulations to control diseases caused by *Pythium* species. Diseases caused by *Pythium* species are often referred to as *Pythium* blight, grease spot, spot blight, crown rot and root rot. *Pythium* also causes seedling disease. All turf grasses are susceptible to attack by *Pythium* spp., including creeping bentgrass, the most popular grass for golf course greens, and the new types of bentgrass grown in the southern United States. Resistant biotypes of *Pythium* typically do not respond to traditional fungicide application. The saponin-containing formulations typically are applied as a foliar spray prior to or upon the first appearance of conditions that promote growth of *Pythium* species. For *Pythium* blight, this is particularly when the weather is hot and humid and the nights stay warm.

Another preferred use of the methods and compositions of the invention is to control *Rhizoctonia* blight, commonly referred to as brown patch. *Rhizoctonia* blight is an especially rapid and destructive disease when environmental conditions favor its growth. Thus the formulations to be used generally should be applied when there is hot, humid weather, high nitrogen fertilization rates, dense foliar growth and/or frequent watering. *Rhizoctonia* blight control on turfgrasses is difficult and the traditional fungicides often fail for this disease. Control of *Rhizoctonia* blight of St. Augustine grass using the subject formulations also is of interest.

Of particular interest is the treatment and prevention of thrip, powdery mildew, black spot, botrytis and melon aphid infestation of oranamental flowers and other plants targeted by these pests. The subject formulations are of particular use for preventing or treating infestations of rose, christmas trees (*e.g.*, pine), and fruit trees and fruit. For example, the formulations are useful for treating peach against canker and apple against codling moth infestation, and grape from infestation by leaf roller, phylloxera, leaf hopper, botrytis, thrips, and powdery mildew. Preferred formulations include saponin and the aromatic aldehydes of formulae (2) and (5), with formulae (3), (4) and (5) preferred.

An example is the treatment of melon aphid infestation in cotton. The most important species is the cotton or melon aphid (*Aphis gossypii Glover*). Almost as soon as cotton has put out leaves, small, soft-bodied, pale-green plant lice fly to them and start to reproduce. Therefore, the subject formulations should be applied to cotton at an early stage in its development. Foliar spraying is preferred because the aphids feed above ground. The most important time, for treatment is immediately prior to and/or during cool, wet seasons, when the aphids can become sufficiently abundant to stunt and deform the plants.

The subject invention also is useful for treating and preventing late blight. Late blight affects tomato, potato, eggplant and other potato family plants and results from infestation by *Phytophthora infestans*. The subject formulations are preferably applied to the plants during moist periods of mild temperature, which is when pathogenesis generally begins by settling of spores on plant surfaces. Likewise, control of boll weevil (*Anthonomus grandis Boheman*) is also of interest. The injury is caused by the adult weevils and their young or grubs. The adults puncture the squares and bolls by chewing into them. Application of the subject formulation should in particular be directed to this portion (squares and bolls) of the plant.

The saponin-containing formulations also are useful for controlling the time of pollination of flowering plants. For example, to prevent or delay pollination the formulations are applied in an amount sufficient to repel bees and other pollinating insects. By adjusting the residuality of the formulation, the length of time during which pollination is inhibited can be controlled. On the other hand, for plants in which cross-pollination is required for fertilization, application of the formulation during this period should be avoided if the pollinating insect is repelled or killed by the formulation. In particular, pollination by species of *Vespidae,* including social wasps, paper-nest wasps, hornets and yellow jackets, are sensitive to the subject formulations.

Treatment of *Dermaptera* (European Earwigs (*Forticula aureculatia*)) with compounds of the invention also is of interest. Earwigs have extremely wide food tastes and feed on many diverse types of plant material, including flowers, vegetables and fruits, both pre- and post-harvest. Flower blossoms, corn silks (kernels) and new vegetable seedlings are particularly vulnerable. Therefore, the subject formulations are applied, for example, by foliar spraying to the growing plants and/or harvested plant parts sought after by the earwings.

Of particular interest is the postharvest treatment of cut flowers using the formulations of the subject invention to control growth of microorganisms, such as those found in vase solutions. Saponin-containing formulations also can increase the lifespan of cut flowers, which depends in part on control of vase solution microorganisms, such as bacteria and fungi, whenever the stems are in water, and in part on providing a nutrient source for the flavors. Treatment of cut flowers can be done by any number of means suited for a particular purpose, such as dipping the cut stem into a subject formulation of the invention and/or addition of the formulation to a vase solution or by dunking the entire cut flower into the formulation. Examples of organisms which can be controlled postharvest include *Botrytis* blossom blight caused by *Botrytis cinerea* Pers: Fr. on cut flowers, particularly greenhouse-grown roses and many other cut flower crops as well as grapes, gray mold caused by *B. cinerea,* and delay rot on flowers as well as fruit such as raspberries, strawberries, apples, pears caused by *B. cinerea, Rhizopus,* and *Penicillium expansum.* Formulations that contain saponin in combination with compounds of formulas (2) and (5) are preferred. In particular, a formulation that includes saponin, cinnamaldehyde and/or $\alpha$-hexyl cinnamaldehyde, and Tween 80 is preferred. Efficacy of the formulations for promoting or preserving decapitated flower life can be evaluted by monitoring wilting of flowers, leaves, or neck, rehydration. bacterial growth and stem contamination, and postharvest control of microbial and physiological plugging.

The subject compounds of the invention can be used to target the three life stages of codling moth (*Cydia pomonella*), adults, eggs and neonate larvae, before they enter the fruit. For this application, it is necessary to develop a susceptibility profile of these life stages to optimize the timing of application of the subject formulations. This is done by testing the susceptibility of eggs, neonate larvae, and adults to the formulations, both freshly applied the residue that remains after the initial application.

The subject saponin and aromatic aldehyde compositions also are useful for control of San José scale, which is an oddly shaped and immobile insect. Like mealy bugs, scales resemble disease organisms more closely than animals. There are two families of scales: soft scales (*Coccidea*) which tend to feed on garden crops, and the armored scales (*Diaspididae*) that prefer orchard crops. Members of the superfamily *Coccidea*, they attach themselves to leaves, fruit and bark of many different plants. Therefore, the subject formulations are preferably applied to the leaves, fruit and bark of susceptible plants.

The subject methods and compositions are also useful for control of mealybugs, which are similar to aphids, psyllids, and phylloxera. Mealybugs suck the juices from plants and spread disease, and the honeydew they excrete invites the growth of a sooty fungus which interferes with photosynthesis. Foliar application is used to control the sooty fungus, and the amount used should be sufficient to induce SAR in the plant so as to control growth of the sap sucking insects in remote regions of the plant where the insects feed.

In addition to treating a host plant, seeds can also be treated using the subject formulations. The seeds can be dusted with a powder preparation (*see* U.S. Patent Application No. 4,978,686 for examples of inorganic materials to which the formulations can be adsorbed) or admixed in a plant substrate such as vermiculite. Seeds also can be obtained from transgenic crops, wherein the components of formula (1) have been expressed in seed, preferably preferentially in seed. Seedlings grown under sterile conditions from treated seeds are free of susceptible fungi and insects. Additionally, seedlings also can be treated with the subject formulations. In some instances it may be necessary to adjust the treatment formulation so as to reduce any phytotoxicity associated with the treatment as tender young shoots are more likely to exhibit phytotoxicity symptoms.

The following examples are offered by way of illustration and not by way of limitation.

## EXAMPLES

Materials and Methods

The chemicals used in the examples given below were obtained from the following sources: cinnamic aldehyde, Spectrum Chemical Company, NJ; coniferyl aldehyde, APIN Chemical, U.K.; Tween 80 and sodium bicarbonate Spectrum Chemical Company, Gardena, CA; $\alpha$-hexyl cinnamic aldehyde, Firmenich, Plainsboro, New Jersey; saponin, Danco Corp., Fresno, CA.

Example 1

Treatment of Powdery Mildew on Rose Cultivars

A. Potted Roses. Eight cultivars of rose were tested to investigate the effect of a cinnamic aldehyde/$NaHCO_3$ formulation on rust and spores. The cultivars used included Moss unnamed (Moss), Galica, Rosette Delize (Hybrid Tea), Rosa Rugosa Rubra (Rugosa), Abel Morrison (Hybrid perpetual), John Laing, Betty Prior, and Rose de Roi. Five (5) potted cultivars (Moss, Galica, Hybrid Tea, Rugosa, and Hybrid perpetual) were selected and assigned a disease rating after Paulus and Nelson (*supra*) for powdery mildew, rust and spores.[1] The Moss and Galica cultivars were 5

on a scale of 0-5 (where 0=no powdery mildew rust/spores lesions, 1 = 1-25, 2=26-50, 3=51-75, 4=76-90, and 5= >90% total leaves per bush). The Hybrid Tea and Hybrid perpetual were rated 3 and the Rugosa was rated 1. The Moss and Galica also were infected with rust equivalent to a 3 rating.

Each cultivar received a foliar spray of about 100 ml of a cinnamic aldehyde formula containing 5 g cinnamic aldehyde, 80 g $NaHCO_3$, 10g of Tween 80 and water to 1000g. In addition, 250 ml of 0.01% (v/v) aqueous solution of $10^0$ brix saponin extract from the yucca shidigera plant was administered to each potted plant once a week beginning with the date of the first cinnamic aldehyde/$NaHCO_3$ treatment. Control plants received no treatment. A single treatment was eradicative of powdery mildew, rust, and spores through the final weekly field observation eight weeks later as compared to the no treatment controls which remained at disease ratings of 5, 3, and 4 for powdery mildew, rust and spores, respectively. Moreover, the treatment appeared to have induced systemic resistance. No phytoxicity was observed.

B. Field grown roses. Another experiment was designed for field grown cut flower rose to evaluate the efficacy of powdery mildew control by cinnamic aldehyde/$NaHCO_3$ during the same period (season) and environmental conditions. Powdery mildew and rust inoculum were high in the test field, and no additional inoculum was necessary to provide disease pressure. Cultivars John Laing, Betty Prior, and Rose de Roi were used in this investigation. Eight John Laing plants from a block row of sixteen were selected for treatment. Every other plant beginning with the first plant in the row was treated. Three Betty Prior plants selected from a block of six were similarly treated, as were two Rose de Roi plants from a block of four. A single foliar spray treatment (about 100ml) of a formulation containing cinnamic aldehyde (5g), Tween 80 (lOg), $NaHCO_3$ (80g) and water to 1000g was applied to each setting of cultivars. Plants were an average of 0.86 m apart. The disease rating was the same as that used to evaluate powdery mildew in containerized cultivars. Controls were untreated plants. Absence of wind and exact spraying protected controls from spray drift. The John Laing cultivars were young, 45-day-old plants with a rating of 5 for powdery mildew. The Betty Prior cultivars were older (≥240 days), previously sprayed with Eagle (120 days prior) with a rating of 3 for powdery mildew and the Rose de Roi were 240-day-old plants with a rating of 2 for powdery mildew and 2 for rust using the same scale as provided above. Induced systemic resistance was determined by observing the number of lesions of powdery mildew and rust produced on each plant after treatment as compared to untreated controls. Weekly reviews were made of the various plants. The effect of the treatment regimen on growth rate was determined at the last field observation of each plant.

With the exception of the untreated controls and three plants of cultivar Betty Prior which had reinfection of powdery mildew with a rating of 3, all plants were free of powdery mildew at the end of the five week trial. No phytotoxicity was observed. All plants had new growth exceeding that of the untreated controls.

The Mean Percentage of Disease Control (MPDC) was calculated for every group of plants. The results were as follows for powdery mildew: John Laing, 98.3%; Betty Prior, 64.3%; Rose de Roi, 100%. The average for all three roses was 90.7% for powdery mildew. Rust was evaluated only on Rose de Roi, and was 85.0%. Effective fungicides for powdery mildew should provide a MPDC of ≥70% under greenhouse or field conditions, and for rust ≥ 65%.

Potted roses or field grown roses sprayed to runoff with an emulsion containing cinnamic aldehyde and sodium bicarbonate and concomitantly sprayed with saponin remained free of powdery mildew and rust for up to 56 days, while plants sprayed only with water did not. The treated plants also remained free of aphids. It has been reported that induced systemic resistance to powdery mildew of roses sprayed with Rubigon averages about 20 days. Mean disease control determinations of approximately 70% were obtained for roses sprayed with an aqueous solution of cinnamic aldehyde and coniferyl aldehyde or emulsions containing sodium bicarbonate and cinnamic aldehyde and/or coniferyl aldehyde. In parallel experiments, Benomyl gave a mean disease control of approximately 80%.

## Example 2

## Treatment of Fungi and Insects on Roses with Saponin and Coniferyl Aldehyde

Six cultivars of infected rose in dedicated experimental rose gardens are used. Four of Mrs. John Laing (Hybrid perpetual) and two of Marchionese of Londonderry (Hybrid perpetual) are treated with one of two formulations of coniferyl aldehyde. The low dose treatment (T1) is a formulation containing coniferyl aldehyde (5g), Saponin (0.86 ml of a 10° brix saponin solution) (10g), and $H_2O$ for 1000g of product. The high dose treatment (T2) is a coniferyl aldehyde formula comprising of 100g of coniferyl aldehyde, saponin (0.86 ml of a 10° brix saponin solution) and the balance $H_2O$ for 1000g of product. See Table 1 (below).

The plants are assigned a disease rating for powdery mildew and rust using the rating system of Paulus and Nelson (*supra*). Each plant (P1 through P6) receives a ≈ 100ml treatment spray of the formulation as indicated in Table 1 below. Control plants are sprayed with water alone. The change in the rating from pre-treatment to post-treatment is calculated as the mean percentage of disease control (MPDC) as described above.

1. Spores were evaluated only on Moss, Galica and the control plants.

Table 1

| Plant - Treatment/Dose Assignment | |
|---|---|
| Treatment/Dose | Plant |
| T1 - Low<br>T2 - High | P1, P4, P6<br>P2, P3, P5 |

Example 3

Treatment of Powdery Mildew on Rose

A three treatment experiment with cinnamic aldehyde formula, saponin and combined cinnamic and saponin formula is evaluated on field grown roses known to be susceptible to powdery mildew. The plants are blocked by variety before fungicide treatments and are randomized as to the plants. Two varieties are used in each of three experiments as described below. In experiment 1, Reichsprasident von Hindenburg (Bourbon) and Oskar Cordel (Hybrid Perpetual) are used; in experiment 2, Rosa Gallica Officinalis (Apothecary Rose) and Deuil de Paul Fontaine (Hybrid Moss) are used. In experiment 3 Comte de Chambord (Portland) and Madame Pierre Oger (Bourbon) are used. Experiment 1 evaluates the effect of cinnamic aldehyde alone, experiment 2 evaluates the effect of saponin alone and experiment 3 evaluates a combination of cinnamic aldehyde and saponin. Each plant receives a single foliar spray of 100ml following evaluation of powdery mildew using the Paulus/Nelson rating scale (*supra.*). Plants are evaluated using this scale just prior to and four days after treatment.

Example 4

Treatment of Grape Phylloxera with Saponin Alone and/or With Cinnamic Aldehyde or Alpha Hexyl Cinnamic Aldehyde

Feeding Site Location Test

Mortality resulting from physiological process disruption is determined by the adult and nymphal mortality experiment and by the egg hatch experiment. After hatching, new insects must secure a verified appropriate feeding site. This activity must be successful if the life cycle of the insect is to continue. Research indicates that approximately 80% of phylloxera mortality occurs during this activity. Low dose concentrations of formulae may be protective of grape stock roots by disrupting the "search and identify feeding site" behavior of the insect. All three types of effects are evaluated using the following protocols.

Adult and Nymphal Mortality Experiment

Approximately twenty four eggs of phylloxera are allowed to develop for up to 30 days on standard excised grape roots. At around 30 days, some of the insects are nymphs while others are adults. New eggs are removed dunng the process. Insect infected roots are submerged into a test formula for 6 seconds then set aside to dry in the air. The percentage of live insects, as defined by growth, oviposition or limb movement, is determined after 5 days. An insect is considered dead if it abandons its feed site. In an initial test, saponin with doses of 20,000 ppm cinnamic aldehyde in water (*i.e.,* 2% cinnamic aldehyde) or 20,000 ppm alpha-hexylcinnamic aldehyde are evaluated. Water with no additives is used as a negative control, while a positive control solution of 250 ppm malation in water is used.

Egg Hatch Experiment

Mixed age groups of 60 grape phylloxera eggs were established on filter paper (Whatman #1, 5.5 cm circles) in 50x9 mm sealing plastic petri dishes treated with 100 µl of solution. A selected concentration of a test formulation of 400 µl is added to the filter disk and the dish closed with the petri dish cover and placed in a plastic container box. After 6 hours, the box was placed in an environmental chamber at 24°C in the dark. The eggs were placed in groups of 10. After one week, the percentage of hatch was determined. In an initial test, saponin (0.86 ml of a 10° brix saponin solution) with doses of cinnamic aldehyde in 6% $NaHCO_3$ and 2% Tween 80 were evaluated with a single group of eggs at each dosage. Three replicates of the experiment were performed. The effects of the formulation were evaluated after 7 days and scared as the number of nymphs that died in the shell (DIS), or eggs that did not hatch completely (IH) (*i.e.*, all died). LD50 and LD95 are determined by probit analysis. At 100 ppm cinnamic aldehyde, 88% of nymphs

died in the shell and the remaining 12% did not hatch completely. The addition of saponin to the formulation at 100 ppm increased the number of nymphs which died in the shell to 93%. All phylloxera eggs treated with $H_2O$ alone hatched; 100% of those treated with Carbofuran (10 ppm) or malathion (250 ppm) died in the shell.

Grape phylloxera eggs also are treated with saponin (0.86 ml of a 10° brix solution) with or without cinnamaldehyde (CNMA) in a laboratory bioassay. Mortality is indicated by failure to initiate splitting of the egg shell. The 7 day $EC_{50}$ and the $LC_{50}$ for root dip treatments of nymphs and adults after 7 days are evaluated.

Example 5

Protocol for Aphid, Spider Mite and White Fly

Activity of saponin with cinnamic aldehyde and/or coniferyl aldehyde against black bean aphid, *Aphid fabae,* two-spotted spider mite, *Terranychus urticae,* and silverleaf white fly, *Bemisia argentifolii* is determined as follows:

Petri Dish Bioassay

Each petri dish (60 mm diameter) is treated with a specific rate of aromatic aldehyde (*e.g.,* 10-1000 ppm) dissolved in water and/or a serial dilution of a 10° brix saponin extract from *Y. schidigera* (ranging from 0.001%-1% (v/v) aqueous solution), which is then allowed to dry. Twenty specimens of each arthropod are put in each dish, (replicate 10 times). The mortality after three hours in contact with a treated plated, is compared to that of arthropods in petri dishes treated only with diluent.

Plant Foliar Bioassay

Plants are grown in 7.5 mm pot in potting soil in greenhouse. Cotton plants are used for spider mites and white fly and sugar beets are used for aphids. When plants reach 3 leaf stage, they are infested with 60 of the specified arthropod (6 replications). The insect/mite is allowed to settle and feed. The plant is sprayed to runoff with a cinnamic aldehyde or α-hexyl cinnamic aldehyde 100 to 2000ppm, or 0.1 to 2 g/l with a 0.05% to 1% (v/v) aqueous solution of 10° brix saponin extract from Y. *schidigera* with or without a cinnamic aldehyde or α-hexyl cinnamic aldehyde 100 to 2000ppm, or 0.1 to 2 g/l. The plant is covered with tall plastic cage (5 mm tall x 10 mm diameter). The mortality after three days of the insect/mites on the plants sprayed with a test formulation is determined and compared with that of insect/mites on plants sprayed only with water.

Example 6

Treatment of Nematode Infestation

Various kinds of nematodes infest plant tissue, including the stem and bulb nematode *(Ditylenchus dipsaci)* and rootknot nematode *(Meloidogyne spp.*). The treatment of stem nematode *(Ditylenchus dipsaci)* with various formulations containing saponin and/or cinnamic aldehyde is tested as follows.

1. Stem nematodes

Stem nematodes are extracted from garlic cloves by chopping the tissue into a mesh-bottomed beaker and suspending the mesh-bottomed beaker in a beaker of water. Nematodes migrate from the host tissue and sink down through the mesh into the bottom beaker. The supernatant water is removed and the nematodes remaining in the beaker are transferred to a watchglass and used in the treatment protocol as follows. Clear plastic trays are divided into open-topped cells measuring 20 mm x 20 mm x 20 mm. One half ml of tapwater at room temperature (19°C) is pipetted into each cell. Ten nematodes are placed in each cell using an eyelash glued to a dissecting needle to handle each animal. One-half ml of one test solution (0.05% to 1 % v/v aqueous solution of 10° brix saponin extract with or without cinnamic aldehyde or α-hexyl cinnamic aldehyde (100-2000 ppm) is then added to each cell. Water is added to the control wells. Survival of nematodes in the cell is monitored by observation using a binocular microscope. The number of animals surviving 1, 5, 10, 20, 30 and 60 minutes after addition of the solutions is recorded. Mortality is assumed if individual nematodes are immobile and fail to respond to manipulation. The test is repeated three times.

2. Root nematodes

a. Petri dish assay

In a double blind study, concentrations of saponin with or without cinnamic aldehyde were tested for activity against root-knot nematode, *Meloidogyne javanica.* Nematodes were put in direct contact with the formulation and at 24 hour intervals, mortality was assessed both visually and by probing. *Meloidogyne javanica* were produced using hydroponics. The nematodes were harvested and used within 24 hours.

Approximately 100 nematodes in 0.07 mls of water were pipetted into a syracuse dish (Fisher) and 1 ml of test formulation was immediately pipetted into each dish. The dishes were then placed into plastic bags to retain moisture and prevent evaporation. Four syracuse dishes were used for each solution test formulation. Every 24 hours for 7 days, the solutions were examined and the first 10 nematodes encountered were assessed as either living or dead, based on morphological integrity of the nematode and touch. Moving nematodes were counted as living.

At concentrations greater than 100 ppm cinnamic aldehyde in vehicle (2% Tween 80, 6% $NaHCO_3$), 100% nematodes were dead at 24 hours. At 10 ppm, 0%, 15%, 17.5% 22.5%, 27.5%, 52.5% and 52.5% were dead at 24, 48, 72, 96, 108, 132, and 156 hours, respectively. There was no effect on mortality at 1 ppm and 0.1 ppm cinnamic aldehyde in vehicle. Addition of a 1:60 dilution 10 brix concentrate of *Yucca shidigera* saponin resulted in 100% mortality at 24 hours with the lowest concentration of cinnamic aldehyde in vehicle tested, 0.1 ppm. However, saponin alone had the same effect. EtOH (95%) killed all nematodes at 24 hours. Minimal effect of the vehicle on mortality was observed: 2.5% at 72 hours and 5% at 108 hours.

b. Plant Foliar Bioassay.

The subject formulations are tested for ability to reduce grape vine infestation by root knot, ring, stubby root, and roll lesion nematodes. Grape vines (Harmony rootstock) in a vinyard were treated with 1000 ppm or 3000 ppm cinnamaldehyde in a saponin solution (0.86 ml of a 100 brix solution), the commercial anti-nematode agent Nemacur, or a formulation blank. The extent of nematode infestation is determined at the time of treatment and at 30 and 60 days post-treatment.

Example 7

Treatment of Strawberry Red Core (*Phytophthora Fragariae*)

Strawberry red core disease is caused by the fungus *Phytophthora fragariae* Hickman which is spread by means of infected planting material or soil infested with long-lived oospores of infected debris. Various formulations containing saponin and cinnamic aldehyde or alpha hexyl cinnamic aldehyde are tested as follows. Macerated strawberry roots infected with *Phytophthora fragariae* are thoroughly mixed with infested compost and allowed to decompose for 4 to 6 weeks to produce a well rotted inoculum for treatment. This is divided into 1 kg lots and mixed with 1500 ml of a test formulation at different concentrations. After 10 minutes of treatment, the compost is rinsed under running tap water on a 25 mm sieve for a minimum of 5 minutes to remove all traces of the test formulation. The compost is then put into 9-cm diameter plastic pots and planted with 4 strawberry plants per pot. Five pots are used for each treatment. Plants are grown in a controlled environment room at 15°C and 18 h daylength; the compost is kept damp to encourage infection. Pots are placed on grids to avoid cross infection among treatments.

After 9 weeks the strawberry plant roots are washed free of compost and examined for signs of infection by cutting roots longitudinally and looking for red steles, and rotted or brown roots. All infections are confirmed by microscope examination of root pieces for the presence of oospores of *Phytophthora fragariae.*

Example 8

Treatment of Fungal Pathogens on Corn

A three treatment experiment with saponin, with/without cinnamic aldehyde or alpha-hexyl cinnamic aldehyde is evaluated. The amount of saponin used is 0.05% to 1% v/v of an aqueous solution of 10° brix saponin extract from *Y. schidigera.* The formulations are tested on field grown corn known to be susceptible to pathogenic fungal infestation. The plants are blocked by variety before fungicide treatments and are randomized as to the plants. Each plant receives a single foliar spray to run off following evaluation of fungal infection (after Paulus and Nelson). The response variable recorded for each plant is the fungal infection rating based on the Paulus/Nelson *(supra)* rating scale. Plants are evaluated on this scale just prior to and four days after treatment.

Example 9

Pitch Canker Disease

Pitch canker disease, caused by the fungus *Fusarium subglutinans* f. sp. *pini* is characterized by a resinous exudation on the surface of shoots, branches, exposed roots and boles of infested trees. The host and geographic range of the pitch canker pathogen has greatly increased since it was first discovered in California in 1986. The pathogen has recently been discovered in Mexico and Japan.

A double-blind bioassay was undertaken using saponin with and without cinnamic aldehyde in various concentrations and formulations. The bioassay was based on inhibition of radial growth of *Fusarium subglutinans* f. sp. *pini.* Eight ml of formulation (concentration unknown) was pipetted into 200 ml of molten 2 % potato dextrose agar (PDA) and the mixture was dispersed into five plastic petri dishes (25 ml dish). Each of four plates was inoculated at the center with an agar plug transferred from a growing PDA culture of *Fusarium subglutinans* f. sp. *pini* (isolate SL-1, UCB), while the fifth plate was left noninoculated as a control. These steps were repeated for each formulation of the cinnamic aldehyde. As a positive control, four PDA plates amended with 5 ppm benomyl were inoculated as described; the negative control was four untreated PDA plates that were inoculated with *F. subglutinans.* All inoculated and non-inoculated plates were incubated at 18°C for five days, after which colony diameters were measured.

Table 10 shows the colony diameter raw data averages for the bioassay, and Table 11 compares the effect on colony diameter of CNMA at various concentrations, with and without saponin *(Yucca schidegira* extract 10° BRIX at 0.86 ml).

Table 10

| Radial Growth of *Fusarium subglutinans* f. sp. *pini* (Data Averages)* | |
|---|---|
| Treatment | Colony diameter (cm) |
| PDA (unamended) | 4.038 |
| 10 ppm CNMA | 4.363 |
| 100 ppm CNMA | 4.238 |
| 100 ppm CNMA + Saponin | 4.300 |
| Glutaraldehyde 2% | 3.663 |
| 5,000 ppm CNMA + Saponin | 2.913 |
| 10 ppm CNMA + Saponin | 3.600 |
| $H_2O$ | 3.738 |
| 2,500 ppm CNMA | 3.513 |
| 2,500 ppm CNMA + Saponin | 3.600 |
| Saponin | 4.138 |
| 5,000 CNMA | 2.908 |
| Formula Blank | 4.380 |
| 12,500 CNMA + Saponin | 0.000 |
| 25,000 CNMA | 0.000 |
| 5 ppm Benomyl (Positive Control) | 0.000 |
| 12,500 ppm CNMA | 0.000 |
| 25,000 CNMA + Saponin | 0.000 |

Table 11

| Radial Growth of *F. subglutinans* f. sp. *pini* | | |
|---|---|---|
| Treatments | | |
| ppm | CNMA colony diameter (cm) | CNMA + Saponin (.86 ml) colony diameter (cm) |
| 10 | 4.36 | 3.60 |
| 100 | 4.24 | 4.30 |

Table 11 (continued)

| Radial Growth of *F. subglutinans* f. sp. *pini* | | |
|---|---|---|
| Treatments | | |
| ppm | CNMA colony diameter (cm) | CNMA + Saponin (.86 ml) colony diameter (cm) |
| 2,500 | 3.51 | 3.60 |
| 5,000 | 2.91 | 2.91 |
| 12,500 | 0 | 0 |
| 25,000 | 0 | 0 |
| Controls | colony diameter (cm) | |
| 2% Glutaraldehyde | 3.66 | |
| $H_2O$ | 3.74 | |
| 5 ppm Benomyl | 0 | |
| PDA (unamended) | 4.04 | |

Example 10

Synergist bioassay

The synergistic effect of a compound is determined following the protocols described herein. An effective amount of a synergist test compound is added or applied separately in combination with a given formulation in a serial dilution and concentration regime ranging from control levels devoid of the synergist test compound to a concentration that is well above optimum. The antipathogenic and/or phytotoxic effect of a particular synergist test compound on a given pathogen and/or plant host is measured for each formula and component with or without a serial diluent of the synergist test compound following essentially the protocols of the above Examples. Optimal dose-range is calculated *in vitro* and *in vivo* from control dose levels to saturation dose levels in which a further increase in one controlling variable produces no further positive increase in the resultant effect. The effectiveness of each test formulation is measured by comparing to control formulations lacking of the synergist test compound and MDICs and MDITs calculated. Synergist compounds are identified which provide: a mean disease resistance for a particular formulation of at least about 60% or better with an optimum of about 70% or greater; and/or a phytotoxicity rating of 2 or less, with 1 or less being optimum.

Saponin bioassay

A saponin or saponin extract is admixed or applied as a separate solution in combination with a given formulation in a serial dilution regime ranging from control levels with 0% saponin and 100% other component through equal and then saturation level of each component. The synergistic effect of the formulation is measured by examining the antipathogenic and phytotoxic effect of a particular pathogen and/or plant host to each formula and component with or without a serial diluent of saponin following the protocols of the above Examples. An effective amount of saponin is measured by comparing to an equivalent in activity of a saponin extract control from *Y. schidigera* comprising 0.05% to 1% v/v aqueous solution of 10° brix saponin extract. Optimal dose-range for a given formulation is calculated *in vitro* and *in vivo.* The effectiveness of each test formulation is measured by comparing to formulations devoid of saponin. Saponins having synergistic properties are identified which provide: a mean disease resistance for a particular formulation of at least about 60% or better with an optimum of about 70% or greater; and/or a phytotoxicity rating of 2 or less, with 1 or less being optimum.

Example 11

Effect of Saponin and/or Cinnamic Aldehyde on Snails

Materials and Methods

Experiment 1:

Contact molluscicidal bioassay studies were carried out by a substrate contact method. For liquid formulation,

filter paper discs (15 cm in diameter) were dipped in test solutions for 2 seconds and subsequently air dried. For dry formulations, substrates were placed on filter paper discs to a depth of 0.5 cm. Dry substrate (*e.g.*, saponin fiber, mill run) were wetted with 10 ml of formula. Discs were placed on the bottom of paper plates (25 cm diameter) the rims of which had been coated with petroleum jelly and NaCl to prevent animal escape from plates. Four snails (*Sephia honensis*) from Connecticut Valley Biological and two slugs (*Deroceras reticulatum*) from Carolina Science were placed on the treated or piled discs in each replication (five replications). Experimental formulations were assigned as:

| Formulation and Number | Composition |
|---|---|
| #1 | F1 - 2% Cinnamic Aldehyde, 2% T80, 6% $NaHCO_3$ |
| #2 | F2 = Saponin (10° BRIX) and F1 (1:9) |
| #3 | F3 = Saponin Fiber + F1 (to wet - 10 ml) |
| #4 | F4 = Saponin Fiber + Wheat Mill Run (50/50 w) |
| #5 | F5 = Saponin (10° BRIX) (to wet - 10 ml) and Wheat Mill Run |
| #6 | F6 = Saponin Fiber |
| #7 | F7 = Formula Blank (CNMA) (to wet - 10 ml) and Wheat Mill Run |
| #8 | F8 = $H_2O$ |
| #9 | F9 = Lilly Miller "SSIKB" (Positive Control - Metaldehyde product) |
| #10 | F10 = CNMA (encapsulated) in oil (Calgene cc-22F) emulsion, encapsulated CNMA @ 31.4+ 1.2% w/w in starch sucrose shell (85/15) |

Experiment 2:

The cinnamic aldehyde formula components were evaluated for activity as follows. Filter paper discs 7.5 cm in diameter were wetted completely with product or component and allowed to air dry. Discs were placed in bottom of "Mason 1/2 pint" glass jars. Two snails and one slug were put in each jar for 5 replications. Jars were sealed with cheese cloth and lid holder to permit respiration. Observations were made at 48 hrs.

Results and Discussion

In general, as shown in Table 12, the formulations containing cinnamic aldehyde were effective molluscicides demonstrating 100% mortality at 48 hrs. The F1 formula performed as well as a saponin-based formula and had a higher percent mortality than the positive control (F9-"SSIKB"). Crude saponin fiber (F3-with F1) combined with CNMA achieved a high percent mortality early in the experiments, second only to encapsulated CNMA which was extremely lethal achieving 100% mortality in 30 minutes. Table 13 presents data from experiment 2 components for formula, negative control and related activity percentages.

Experiment 3:

Saponin (0.86 ml 10° brix solution) with or without (10-25,000 ppm) is cinnamic aldehyde evaluated for effect on moss dessication. A Whatman filter paper disc (7.5 cm) is placed on the bottom of each petri dish. Three ml of $H_2O$ is pipetted onto the discs. Moss sections (3.5 cm x

Table 12

| Data Analysis - Slugs and Snails | | | | | | | |
|---|---|---|---|---|---|---|---|
| | Formula | | % Mortality @ Hrs. After Treatment | | | | |
| | | | 0 | .5 | 2.5 | 24 | 48 |
| #1 | F1 (2%) | Snails<br>Slugs | 0<br>0 | 0<br>0 | 50<br>100 | 50<br>100 | 100<br>100 |
| #2 | 10°B+ FI | Snails | 0 | 0 | 25 | 50 | 50 |

Table 12 (continued)

| Data Analysis - Slugs and Snails | | | | | | | |
|---|---|---|---|---|---|---|---|
| | Formula | | % Mortality @ Hrs. After Treatment | | | | |
| | | | 0 | .5 | 2.5 | 24 | 48 |
| | | Slugs | 0 | 0 | 50 | 100 | 100 |
| #3 | S.FIBER/F1 | Snails<br>Slugs | 0<br>0 | 0<br>100 | 75<br>100 | 75<br>100 | 100<br>100 |
| #4 | S.FIBER+M.R. | Snails<br>Slugs | 0<br>0 | 0<br>50 | 100<br>100 | 100<br>100 | 100<br>100 |
| #5 | 10°B+M.R. | Snails<br>Slugs | 0<br>0 | 0<br>0 | 50<br>0 | 75<br>100 | 100<br>100 |
| #6 | S.FIBER | Snails<br>Slugs | 0<br>0 | 0<br>0 | 75<br>100 | 75<br>100 | 75<br>100 |
| #7 | CNMA BLANK+M.R. | Snails<br>Slugs | 0<br>0 | 0<br>0 | 0<br>0 | 0<br>100* | 0<br>100* |
| #8 | NEG.CONT. ($H_2O$) | Snails<br>Slugs | 0<br>0 | 0<br>0 | 0<br>0 | 0<br>0 | 0<br>100* |
| #9 | POS.CONT. (SSIKB) | Snails<br>Slugs | 0<br>0 | 0<br>0 | 0<br>0 | 50<br>100 | 75<br>100 |
| #10 | CNMA ENCA+OIL | Snails<br>Slugs | 0<br>0 | 100<br>100 | 100<br>100 | 100<br>100 | 100<br>100 |

* Animal Contacted Plate Rim (NACL)

3.5 cm) are taken from the core of potted stock and placed on the filter paper discs for all plates. Two ml of test solutions are sprayed on each dish, with five replicates per treatment. The petri dishes are left at ambient temperatures and areas of desiccation are observed at 24, 48 and 60 hours post-treatment. The results are shown in Table 13. In general, moss treated with the cinnamic aldehyde formulation showed the greater percentage of dessication over time. While saponin was effective both alone and in combination with cinnamic aldehyde, it did increase the amount of desiccation over that seen with cinnamic aldehyde alone.

The data in Table 14 shows the percent activity from formula components.

Example 12

Phytotoxicity and Bioassay of Formulations Containing Cinnamic Aldehyde and Saponin

A. Phytotoxicity Trials

Phytotoxicity trials were performed on three greenhouse crops to determine the compatibility of using saponin as surfactant adjuvant with CNMA in place of polysorbates (*e.g.* Tween). The following summarizes assay results:

Table 13

| BRYOPHYTA (MOSS) Percent of Desiccation (over time) | | | | | |
|---|---|---|---|---|---|
| | | 0 | 24 hrs. | 48 hrs. | 60 hrs. |
| MOSS:<br>DICRANUM | F1<br>F2<br>F3<br>F4<br>F5 | 0<br>0<br>0<br>0<br>0 | 15<br>10<br>15<br>5<br>6 | 40<br>40<br>50<br>15<br>18 | 90<br>80<br>85<br>25<br>26 |

Table 13 (continued)

| BRYOPHYTA (MOSS) Percent of Desiccation (over time) | | | | | |
|---|---|---|---|---|---|
| | | 0 | 24 hrs. | 48 hrs. | 60 hrs. |
| SPAGNUM (BOG MOSS) | F1 | 0 | 20 | 60 | 90 |
| | F2 | 0 | 15 | 55 | 75 |
| | F3 | 0 | 20 | 60 | 85 |
| | F4 | 0 | 8 | 12 | 20 |
| | F5 | 0 | 9 | 12 | 22 |
| WOODLAND: | F1 | 0 | 20 | 60 | 85 |
| | F2 | 0 | 10 | 40 | 70 |
| | F3 | 0 | 20 | 50 | 80 |
| | F4 | 0 | 10 | 15 | 20 |
| | F5 | 0 | 12 | 18 | 28 |
| F1 CNMA 2%<br>F2 SAP (10° Brix)<br>F3 CNMA 2% + SAP<br>F4 -CONTROL $H_2O$<br>F5 Formula Blank | | | | | |

Table 14

| MOSS (60 hrs) | |
|---|---|
| Formulation | % Desiccation |
| None | 70 |
| T80 | 10 |
| $NaHCO_3$ | 20 |
| T80+$NaHCO_3$ | 25 |
| Formula | 90 |
| Neg. Control | 10 |

1. Mini roses, (Sunburst). Four potted minirose plants (Sunburst) were treated with each of three treatment applications; 0.5% CNMA plus 0.05% saponin, 0.25% CNMA plus 0.025% saponin and a water only control. Plants were sprayed in the laboratory using a spray tower, all plants were sprayed to runoff. After spraying, plants were observed for a period of five days. No phytotoxicity was observed on old growth, new growth or on flower petals, indicating these rates are safe for applying to miniroses.

2. Chrysanthemums. Three potted chrysanthemums each were treated with 0.5% CNMA plus 0.05% saponin, 0.25% CNMA plus 0.025% saponin, or a water only control. Plants were sprayed as discussed above. After a five-day observation period no phytotoxicity was observed on leaf or flower petals, demonstrating these rates are safe for application.

3. Poinsettias. Two potted poinsettias each were treated with 0.5% CNMA plus 0.05% saponin, 0.25% CNMA plus 0.025% saponin, or a water only control. After a five-day observation period, phytotoxicity was observed on new leaf growth of the high application rate (0.5% CNMA, 0.05% saponin). No symptoms were observed on new leaf growth of the lower rate (0.25% CNMA, 0.025% saponin), indicating the lower rate is safe for application.

B. Pest Insect Bioassays

1. Two-spotted Spider Mites. Mites were assayed by placing rose leaves infested with spider mites in approximately equal numbers in petri dishes with Whatman paper placed on the bottom. Four petri dishes with mites were sprayed on both sides of leaves for each of three treatments: 0.5% CNMA plus 0.05% saponin, 0.25% CNMA plus

0.025% saponin and a water only control. Treated mites were left for 24 hours and the number of surviving mites were then counted and recorded. Results were as follows: Control petri dishes ($H_2O$ only), 53.25 ± 15.57 (mean±SE); 0.25% CNMA, 6.75 ± 1.18; 0.5% CNMA, 0.75 ± 0.48. These results indicate that formulations containing CNMA plus saponin have a high degree of efficacy against mites for direct spray applications.

2. Western Flower Thrips. Thrips were assayed by placing rose leaves infested with thrips in approximately equal numbers in petri dishes with Whatman paper placed on the bottom. Four petri dishes with thrips were sprayed on both sides of leaves for each of three treatments: 0.5% CNMA plus 0.05% saponin, 0.25% CNMA plus 0.025% saponin and a water only control. Treated thrips were left for 6 hours and the number of dead thrips were then counted and recorded. Results were as follows: Control petri dishes ($H_2O$ only), 1.4% ± 0.85% (mean SE); 0.25% CNMA, 53.2% ± 11.8; and 0.5% CNMA, 87.2% ± 2.79. These results indicate that CNMA plus saponin have a high degree of efficacy against thrips for direct spray applications.

3. Melon Aphids. Melon aphids were assayed using whole, nonflowering potted chrysanthemum plants. Two plants were treated for each treatment and two leaves, one from the top of the plant and one from the bottom of the plant, were sampled to determine the number of living and dead melon aphids. Three treatments were applied: 1.0% CNMA plus 0.5% saponin, 0.5% CNMA plus 0.25% saponin, and 0.5% saponin only. The whole plants were sprayed to "drip" on both the top and bottom sides of leaves. Results are presented as the proportion of aphids found dead. Results were as follows: control plant (0.5% saponin only) 14.8% ± 4.5; 0.5% CNMA 48.3 ± 16.1; 1.0% CNMA 72.0% ± 11.2. These results indicate the CNMA can kill a high degree of aphids with direct applications.

Residual Activity of Saponin Cinnamaldehyde and α-hexyl Cinnamaldehyde

Two separate experiments indicated that both cinnamaldehyde (CNMA) and α-hexyl cinnamaldehyde (HCA) have residual activity. In the first experiment, two ml of two concentrations of CNMA (0.3 and 1 %) were sprayed on filter paper (Whatman). As a negative control, two ml of water was also sprayed on filter paper. Twenty-four hours later, two ml of water were sprayed on treatment and control filter paper, which were then dried for 30 min. Approximately 30 thrips insects (*Frankliniella occidentalis*) were introduced and the number of *F. occidentalis* were observed after one hour. Mean mortality was calculated for each treatment.

After 72 hours, the treatment filter papers were flipped over and only the negative control filter paper and the filter paper treated with 1 % CNMA were sprayed with 2 ml of water and allowed to dry for 30 minutes. Approximately 30 thrips were introduced to the two treated filter papers and after one hour the number of dead F. *occidentalis* were observed and the mean mortality calculated for each treatment. A similar assay was conducted using α-hexyl cinnamaldehyde. Mean mortality was higher for rehydrated filter papers compared to non-rehydrated filter papers over time. These experiments demonstrate that rehydration plays a role in the continued lethal effects of treated filter paper in contact with thrips.

Continuous Exposure Tests

To further determine the residual activity of saponin, CNMA and HCA, insects are confined to deposits on two representative surfaces. Glass is used to represent nonporous surfaces and filter paper is used as a porous surface. Two ml of five different concentrations of saponin with or without an aromatic aldeltyde in a formula are applied to filter paper disks (9 cm diameter) or the bottoms of glass petri dishes (9 cm diameter). As a control, two ml of formula minus saponin are also applied. The deposits are allowed to dry for 24 hours before testing. At test intervals of 7, 14, 28, and 56 days, one set of plates and filter papers are rehydrated with 2 ml of water, while a parallel set is not rehydrated. Insects are then confined to the deposits continuously and the number of insects killed by the deposits is counted regularly. If deposits fail to kill insects within 48 hours, these treatments are discontinued from further aging studies.

Example 14

Overproduction of Aromatic Aldehydes in Transgenic Plants

Twenty μg of polyA RNA is prepared from a plant tissue that produces cinnamaldehyde, and cDNA is synthesized. Part of this is cloned into lambda-ZAP II vector (a commercially available cloning vector). At least 500,000 recombinants are screened using an oligonucleotide probe designed from conserved sequences of cloned CA4H and CAD genes obtained from GenBank, or designed from peptide sequence of purified protein from the intended host plant. Strongly hybridizing clones are selected and used to rescreen the cDNA library. The resulting clones are sequenced to enable the introduction of appropriate gene sequences into a plant expression cassette in either antisense or sense orientation. The antisense and sense constructs are introduced into *Agrobacterium tumefaciens* LBA4404 by direct transformation following published procedures. Tobacco (*N. tabacum* variety Samsun) leaf discs are transformed using well estabished published procedures (Horsch *et al.* (1985) *Science* 227:1229-1231. Plants containing either CA4H or CAD constructs are identified by PCR or hybridization and selected for further analysis.

Plant material from both transformed and untransformed control plants is used for determinations of CA4H and CAD enzyme activity using well established published assays. Plants in which the activity of CA4H or CAD has been reduced to less than 20% of that seen in control plants are selected for further analysis. Selected plants with low CA4H activity are crossed with plants with low CAD activity and progeny inheriting both gene constructs are selected by PCR. Plants with suppressed CA4H and suppressed CAD activity are analyzed for aromatic aldehyde production using standard pub!ished procedures.

Example 16

Production of Aromatic Aldehydes in Microbial Systems

A cDNA library is generated using RNA extracted from six week old tobacco stems. 20μg of polyA RNA is prepared and cDNA synthesized. Part of this is cloned into lambda-ZAP II vector (a commercially available cloning vector). At least 500,000 recombinants are screened using an oligonucleotide probe designed from peptide sequence sequences of CCoAr protein purified from six week old tobacco stem tissue using the protocol of Goffner, et al., *Plant Physiol.* (1994) 106:625. Strongly hybridizing clones are selected and used to rescreen the cDNA library. The resulting clones are sequenced to enable the identification of full-length cDNA inserts and the introduction of appropriate CCoAR gene sequences into yeast expression vector pMTL8110 (Faulkner, et al (1994) *Gene* 143:13-20. The coding sequences for *Rhodosporidium toruloides* phenylalanine ammonia lyase (PAL; GenBank locus RHDPAL) and a parsley 4-coumarate:CoAl ligase (4CL; GenBank locus PC4CL1AA) are similarly introduced into equivalent yeast expression vectors. The PAL,4CL and CCoAR constructs are used to transform *Saccharomyces cerevisiae* strains by electroporation using established published procedures (Becker, and Guarente, *Methods in Enzymology* 194:182-187, 1991; Simon, (1993) *Methods in Enzymol* 217:478-483. Transformants are selected on minimal medium lacking leucine. Transformant strains carrying all three gene constructs are identified by PCR and selecter for further analysis.

Extracts from both transformed and untransformed control strains are used for determinations of PAL, 4CL and CCoAR enzyme activities using well established published assays. Strains in which the activity of PAL, 4CL and CCoAR is significantly greater than the background activity detected in control strains are selected for further analysis. Selected strains are analyzed for aromatic aldehyde production using standard published procedures and those producing significant amounts of cinnamaldehyde are selected for optimization of fermentation conditions.

Example 17

Construction of a Yeast Strain that Produces HCA

A yeast strain, such as *Saccharomyces cerevisiae,* which contains the enzymes necessary for biosynthesis of cinnamaldehyde (CNMA) is constructed as follows. First, the strain is engineered for high-level expression of PAL as described by Faulkener *et al.* (1994) *Gene* 143:13020. A plant cinnamate 4-hydroxylase gene is also operably linked to appropriate control signals and inserted into the strain (*see* Urban *et al.* (1994) *Eur. J. Biochem.* 222:843-850). A cinnamoylCoA reductase (CCoAR) gene is obtained by applying standard gene cloning techniques to isolate a cDNA clone using as a probe a nucleotide sequence derived from a partial amino acid sequence of the purified protein, which has been purified and partially characterized from several plant sources. The CCoAR gene is also linked to control signals operable in yeast and inserted into the yeast strain.

The gene encoding the enzyme that catalyzes the conversion of CNMA to HCA is then cloned as follows. A cDNA library is constructed in a yeast expression vector using mRNA obtained from rice plants. The cDNA library is then transformed into the previously constructed yeast strain and transformants selected using a selectable marker present on the expression vector.

To identify those yeast strains that produce HCA, transformants are transferred to microtiter wells containing yeast growth medium agar. Microtiter plates are then placed in a chamber that contains fleas, which are sensitive to HCA but not to CNMA. Yeast strains from wells that contain a statistically significantly greater number of dead fleas than wells containing untransformed control yeast strains are diluted and re-plated in microtiter plates, after which the screening process is repeated to obtain colonies derived from a single transformed yeast cell.

Single cell-derived colonies that exhibit increased flea mortality are analyzed for HCA production by gas liquid chromatography (GLC), using a 30 meter non-polar polydimethylsiloxane capillary column (e.g. HP-1, Hewlett-Packard, or SPB-1, Supelco) and a flame-ionization detector. Using helium as a carrier gas (8 ml/min.) and a column temperature of approximately 240°C, the (E)-cis isomer (major component) has a retention time of approximately 6.0 minutes and the (Z)-trans isomer (minor component) has a retention time of approximately 6.3 minutes.

Expression vector DNA is isolated from colonies that produce HCA and the insert sequenced to obtain the nucleotide sequence and deduced amino acid sequence of the enzyme that catalyzes the conversion of CNMA to HCA.

Example 18

Construction of Transgenic Plants that Produce HCA

A gene that codes for the enzyme that catalyzes the conversion of cinnamic aldehyde (CNMA) to α-hexyl cinnamic aldehyde (HCA) is cloned from rice plants by transposon mutagenesis. Rice protoplasts are transformed with a cloning vector that contains a maize Ac transposable element inserted into a hygromycin B phosphotransferase (*hyg*B) gene so as to disrupt the coding sequence of the *hyg*B gene (*see, e.g.,* Izawa *et al.* (1991) *Mol. Gen. Genet. 227:* 391-396; Murai *et al.* (1991) *Nucl. Acids. Res.* 19: 617-622). Transformed protoplasts are plated on growth medium agar containing sufficient amounts of hygromycin B to prevent non-hygromycin resistant protoplasts from regenerating. Protoplasts in which the Ac transposable element has jumped from the *hyg*B gene to the rice genome are resistant to hygromicin B, and thus regenerate to form callus tissue. Plants are regenerated from hygromycin-resistant callus tissue (*see,* Izawa *et al., supra.).*

Regenerated rice plants are analyzed for the presence or absence of CNMA and HCA as described in the previous Example. Plants that produce CNMA but not HCA potentially carry an AC transposon inserted into the HCA biosynthesis gene. Genomic DNA is isolated from $CNMA^+/HCA^-$ mutants and hybridized to labeled transposon DNA. Fragments that hybridize to the transposon DNA probe are subcloned into an appropriate cloning vector for mapping and sequence analysis. The HCA biosynthesis gene is identified as an open reading frame that is disrupted by insertion of the known sequence of the Ac transposon. A fragment of the gene is used to probe a cDNA library to isolate the corresponding cDNA.

The cDNA for the HCA biosynthesis enzyme is inserted into an expression vector, operably linked to a strongly expressed promoter that is functional in plant for which pest resistance is desired. The expression vector is inserted into the plants using transformation methods known to those of skill in the art. Transgenic plants are analyzed for HCA production and/or repellant or pesticidal activity against pests as described in Example 17 above.

As the above results show, potted roses or field grown roses sprayed to run off with an emulsion containing cinnamic aldehyde and sodium bicarbonate and concomitantly sprayed with saponin remained free of powdery mildew and rust for up to 56 days as compared to plants sprayed only with water. The plants also remained free of aphids. The results also demonstrate that saponin increases the effectiveness of cinnamic aldehyde against pests such as phylloxera, nematodes, slugs, snails, and fungi such as *Fusarium subglutinans.* Saponins and CNMA formulations were also demonstrated to be effective against spider mites, thrips, and melon aphids. The invention is suited to provide seeds, seedlings, plants, and plant parts such as fruit substantially free of pathogenic organisms such as fungi and sapsucking insects. The invention further is suited for reducing the level of mycotoxins and other toxic secondary metabolites associated with plant parts such as stems, leaves, roots, fruit, seeds, and/or flowers before, during and/or after the plant and/or plant part is harvested and/or processed for consumption.

All publications and patent applications mentioned in this specification are indicative of the level of skill of those skilled in the art to which this invention pertains.

## Claims

**1.** A method for controlling growth of pathological organisms on a plant whereby the plant surface is provided with an aqueous composition comprising an effective pathological organism growth controlling amount of one or more saponin compounds and at least one compound according to the following formula

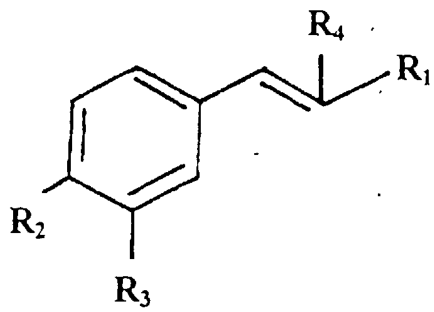

(2)

wherein $R_1$ represents -CHO, $R_2$ represents H, -OH or an organic substituent comprising 1-10 carbon atoms, $R_3$ represents -H, $-OCH_3$ or an organic substituent comprising 1-10 carbon atoms and $R_4$ represents -H or an organic substituent comprising 1-10 carbon atoms, whereby said amount is nonphytotoxic to said plant.

**2.** A method according to claim 1, whereby said plant is contacted with an aqueous formulation comprising 0. 01-50 g/l of one or more of the compounds according to the formula (2) of Claim 1.

**3.** The method according to Claim 1 whereby said aqueous formulation comprises 0.025 - 3% of saponin.

**4.** A method according to claim 1 or 2, wherein $R_1$ represents -CHO, $R_2$ and $R_3$ represents -H and $R_4$ represents -$(CH_2)_5$-$CH_3$.

**5.** A method according to anyone of the afore going claims wherein the plant species is rose, grape, apple, peach, turfgrass, tomato, bell pepper, pine or cotton species.

**6.** A method according to anyone of claims 1-5, whereby pathological organisms comprise at least one of fungi, bacteria, nematodes, algae, insects, arachnids and terrestrial mollusks.

**7.** A method according to claims 5 and 6 whereby'the pathological organism is a fungus causing powdery mildew, rust, botrytis, pitch canker, *Sclerotonia* dollarspot, *Phythium* blight and *Rhizoctonia* blight.

**8.** A method according to anyone of claims 1-6, whereby the pathological organism is at least one of aphids, leaf hopper, leaf rollers, stem and bulb nematodes, phylloxera, blue-green grass algae, condling moth and thrips.

**9.** A method according to anyone of claims 1-3 and 5-8 wherein at least one of the compounds is cinnamic aldehyde or coniferyl aldehyde.

**10.** An aqueous composition for use in a method according to any one of the aforegoing claims comprising one or more saponin compounds and 0.01-50 g/l of one or more compounds of the formula

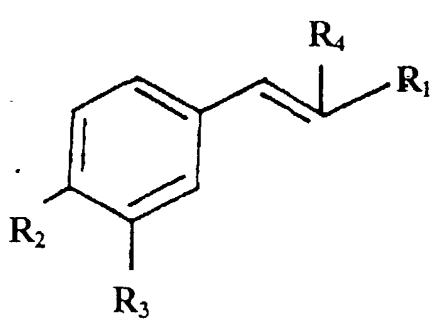

(2)

wherein $R_1$ represents -CHO, $R_2$ represents -H, -OH or an organic substituent comprising 1-10 carbon atoms, $R_3$ represents -H, $-OCH_3$ or an organic substituent comprising 1-10 carbon atoms, and $R_4$ represents -H or an organic

substituent comprising 1-10 carbon atoms.

**11.** A composition according to Claim 10 wherein $R_1$ represents -CHO, $R_2$ and $R_3$ represent -H and $R_4$ represents -$(CH_2)_5$-$CH_3$.

**12.** The composition according to Claim 10 or 11, wherein said saponin is in an amount of 0.025 - 3%.

**13.** A composition according to claim 10 wherein at least one compound is cinnamic aldehyde or coniferyl aldehyde.

**14.** Use of a composition according to any one of claims 10-13 in controlling growth of pathological organisms on a plant.

**15.** A method for inducing resistance of plants against pathological organisms whereby said plants are contacted with an amount of a combination of at least one saponin compound and at least one compound of the formula

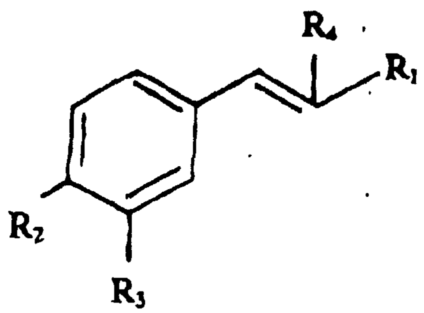

(2)

wherein $R_1$ represents -CHO, $R_2$ represents -H, -OH or an organic substituent comprising 1-10 carbon atoms, $R_3$ represents -H, $-OCH_3$ or an organic substituent comprising 1-10 carbon atoms, and $R_4$ represents -H or an organic substituent comprising 1-10 carbon atoms whereby said amount is sufficient to induce systemic resistance to said pathological organisms.

**16.** An aqueous composition for use in a method according to Claim 15, comprising at least one saponin compound and at least one compound of the formula

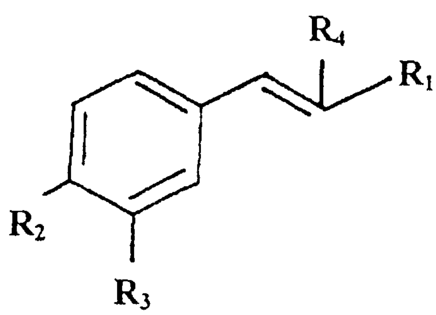

(2)

wherein $R_1$ represents -CHO, $R_2$ represents -H, -OH or an organic substituent comprising 1-10 carbon atoms, $R_3$ represents -H, $-OCH_3$ or an organic substituent comprising 1-10 carbon atoms, and $R_4$ represents -H or an organic substituent comprising 1-10 carbon atoms in an effective amount to induce said systemic resistance.

**17.** Use of a composition according to claims 10-13 or 16 in inducing systemic resistance in plants against pathological organisms.

**18.** An aqueous composition comprising a pathogen growth modulating amount of at least one saponin compound and one or more compounds of the formula

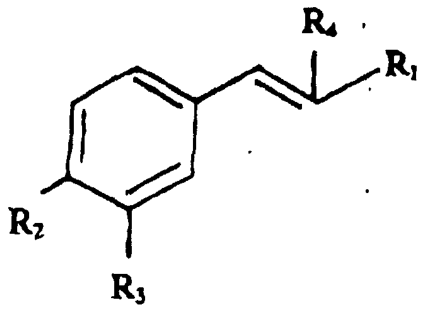

(2)

wherein $R_1$ represents -CHO, $R_2$ represents -H, -OH or an organic substituent comprising 1-10 carbon atoms, $R_3$ represents -H, $-OCH_3$ or an organic substituent comprising 1-10 carbon atoms, and $R_4$ represents -H or an organic substituent comprising 1-10 carbon atoms, in an agriculturally compatible carrier, wherein said composition provides a mean disease resistance of 70% or higher against at least one pathogenic organism colonizing one or more plant surfaces.

**19.** Use of a composition according to claims 10-13, 16 or 18 for providing a mean disease resistance of 70% or higher against at least one pathogenic organism colonizing one or more plant surfaces.

**20.** A method for screening for sensitivity of a plant pathogen to a fungal growth modulating formulation comprising at least one saponin compound and at least one compound of the formula

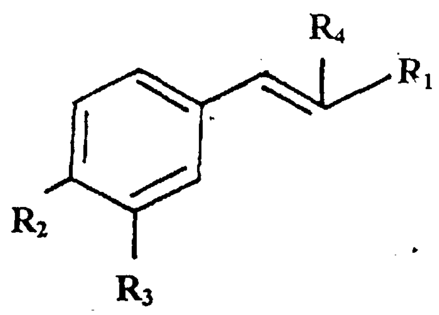

(2)

wherein $R_1$ represents -CHO, $R_2$ represents -H, -OH or an organic substituent comprising 1-10 carbon atoms, $R_3$ represents -H, $-OCH_3$ or an organic substituent comprising 1-10 carbon atoms, and $R_4$ represents -H or an organic substituent comprising 1-10 carbon atoms, said method comprising:

contacting said plant pathogen with said fungal growth modulating formulation; and determining the efficacy of said formulation as a fungal pathogen.

**21.** A method for controlling growth of pathological organisms on a plant whereby the plant surface is provided with an aqueous composition comprising one or more saponin compounds and at least one compound according to the following formula

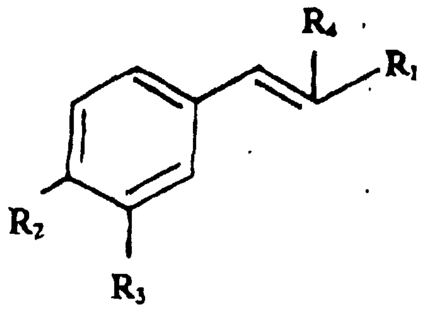

(2)

wherein $R_1$ represents -CHO, $R_2$ represents -H, -OH or an organic substituent comprising 1-10 carbon atoms, $R_3$ represents -H, $-OCH_3$ or an organic substituent comprising 1-10 carbon atoms, and $R_4$ represents -H or an organic substituent comprising 1-10 carbon atoms, whereby said saponin compounds are synergists to increase the effect of said compound of formula (2) on controlling growth of pathological organisms on a plant and allow for a reduction in the concentration of said compound of formula (2) in said formulation comparing with the concentration of said compound of formula (2) when used without said saponin compounds.--.

**22.** A method according to claim 21, wherein said formulation is an aqueous formulation comprising 0. 01-50 g/l of one or more of the compounds according to the formula (2) of claim 21.

**23.** A method according to claim 21 or 22, wherein $R_1$ represents -CHO, $R_2$ and $R_3$ represents -H and $R_4$ represents $-(CH_2)_5-CH_3$.

**24.** An aqueous composition for use in a method according to Claims 21-23 comprising one or more saponin compounds and 0.01-50 g/l of one or more compounds of the formula

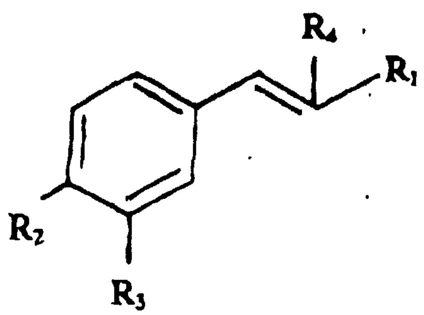

(2)

wherein $R_1$ represents -CHO, $R_2$ represents H, -OH or an organic substituent comprising 1-10 carbon atoms, $R_3$ represents -H, $-OCH_3$ or an organic substituent comprising 1-10 carbon atoms, and $R_4$ represents -H or an organic substituent comprising 1-10 carbon atoms.

**25.** A composition according to claim 24, wherein $R_1$ represents -CHO, $R_2$ and $R_3$ represent -H and $R_4$ represents $-(CH_2)_5-CH_3$.

**26.** A composition according to claim 24, wherein at least one compound is cinnamic aldehyde or coniferyl aldehyde.

### Patentansprüche

**1.** Verfahren zur Bekämpfung des Wachstums pathologischer Organismen an einer Pflanze, wobei die Pflanzenoberfläche mit einer wäßrigen Zusammensetzung versehen wird, die eine effektive, das Wachstum pathologischer Organismen hemmende Menge einer oder mehrerer Saponinverbindungen sowie wenigstens eine Verbindung gemäß der folgenden Formel

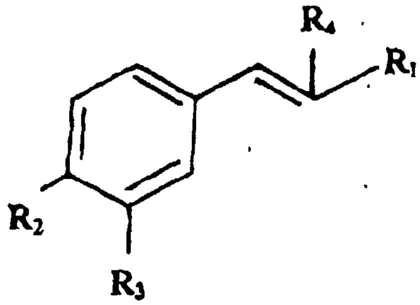

(2)

umfaßt, wobei $R_1$ -CHO darstellt, $R_2$ -H, -OH oder einen organischen Substituenten mit 1-10 Kohlenstoffatomen darstellt, $R_3$ -H, $-OCH_3$ oder einen organischen Substituenten mit 1-10 Kohlenstoffatomen darstellt und $R_4$ -H oder einen organischen Substituenten mit 1-10 Kohlenstoffatomen darstellt, wobei diese Menge für die Pflanze nicht phytotoxisch ist.

**2.** Verfahren gemäß Anspruch 1, wobei die Pflanze mit einer wäßrigen Zubereitung in Kontakt gebracht wird, die 0,01-50 g/l einer oder mehrerer der Verbindungen gemäß Formel (2) von Anspruch 1 umfaßt.

**3.** Verfahren gemäß Anspruch 1, wobei die wäßrige Zubereitung 0,025-3% Saponin umfaßt.

**4.** Verfahren gemäß Anspruch 1 oder 2, wobei $R_1$ -CHO darstellt, $R_2$ und $R_3$ -H darstellen und $R_4$ $-(CH_2)_5-CH_3$ darstellt.

**5.** Verfahren gemäß einem der vorangehenden Ansprüche, wobei es sich bei der Pflanzenart um eine Rosen-, Weinreben-, Apfel-, Pfirsich-, Rasengras-, Tomaten-, Ziegenpfeffer-(Capsicum annuum grossum), Kiefern- oder Baumwollart handelt.

**6.** Verfahren gemäß einem der Ansprüche 1-5, wobei die pathologischen Organismen wenigstens einen Vertreter aus der Gruppe der Pilze, Bakterien, Nematoden, Algen, Insekten, Spinnentiere und landlebenden Weichtiere umfassen.

**7.** Verfahren gemäß den Ansprüchen 5 und 6, wobei der pathologische Organismus ein Pilz ist, der Echten Mehltau, Rost, Grauschimmelfäule (*Botrytis*), Pechkrebs (pitch canker), *Sclerotinia*-Markfleck, *Pythium*-Wurzelfäule und *Rhizoctonia*-Wurzelfäule verursacht.

**8.** Verfahren gemäß einem der Ansprüche 1-6, wobei es sich bei dem pathologischen Organismus um wenigstens einen Vertreter aus der Gruppe der Blattläuse, Zwergzikaden, Wickler, Stengel- und Wurzelälchen, Rebläuse, Grasblaualgen, Apfelwickler und Blasenfüßer (Thrips) handelt.

**9.** Verfahren gemäß einem der Ansprüche 1-3 und 5-8, wobei es sich bei wenigstens einer der Verbindungen um Zimtaldehyd oder Coniferylaldehyd handelt.

**10.** Wäßrige Zusammensetzung zur Verwendung bei einem Verfahren gemäß einem der vorangehenden Ansprüche, umfassend eine oder mehrere Saponinverbindungen sowie 0,01-50 g/l einer oder mehrerer Verbindungen der Formel

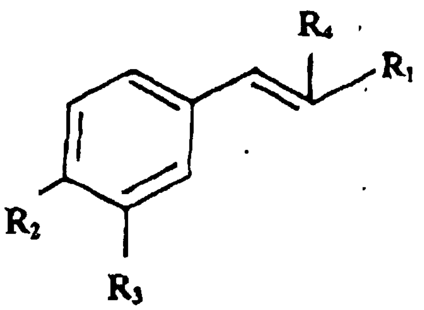

(2)

wobei $R_1$ -CHO darstellt, $R_2$ -H, -OH oder einen organischen Substituenten mit 1-10 Kohlenstoffatomen darstellt, $R_3$ -H, $-OCH_3$ oder einen organischen Substituenten mit 1-10 Kohlenstoffatomen darstellt und $R_4$ -H oder einen organischen Substituenten mit 1-10 Kohlenstoffatomen darstellt.

**11.** Zusammensetzung gemäß Anspruch 10, wobei $R_1$ -CHO darstellt, $R_2$ und $R_3$ -H darstellen und $R_4$ $-(CH_2)_5-CH_3$ darstellt.

**12.** Zusammensetzung gemäß Anspruch 10 oder 11, wobei das Saponin in einer Menge von 0,025-3% vorhanden ist.

**13.** Zusammensetzung gemäß Anspruch 10, wobei es sich bei wenigstens einer Verbindung um Zimtaldehyd oder Coniferylaldehyd handelt.

**14.** Verwendung einer Zusammensetzung gemäß einem der Ansprüche 10-13 bei der Bekämpfung des Wachstums pathologischer Organismen an einer Pflanze.

**15.** Verfahren zur Induktion einer Resistenz bei Pflanzen gegen pathologische Organismen, wobei die Pflanzen mit einer Menge einer Kombination von wenigstens einer Saponinverbindung sowie wenigstens einer Verbindung der Formel

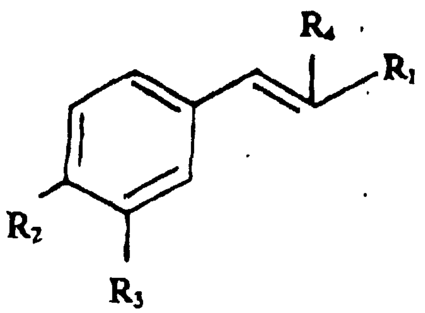

(2)

in Kontakt gebracht werden, wobei $R_1$ -CHO darstellt, $R_2$ -H, -OH oder einen organischen Substituenten mit 1-10 Kohlenstoffatomen darstellt, $R_3$ -H, $-OCH_3$ oder einen organischen Substituenten mit 1-10 Kohlenstoffatomen darstellt und $R_4$ -H oder einen organischen substituenten mit 1-10 Kohlenstoffatomen darstellt, wobei die Menge ausreicht, um eine systemische Resistenz gegen die pathologischen Organismen zu induzieren.

**16.** Wäßrige Zusammensetzung zur Verwendung bei einem Verfahren gemäß Anspruch 15, umfassend wenigstens eine Saponinverbindung sowie wenigstens eine Verbindung der Formel

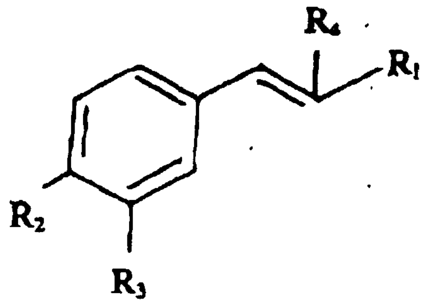

(2)

wobei $R_1$ -CHO darstellt, $R_2$ -H, -OH oder einen organischen substituenten mit 1-10 Kohlenstoffatomen darstellt, $R_3$ -H, $-OCH_3$ oder einen organischen substituenten mit 1-10 Kohlenstoffatomen darstellt und $R_4$ -H oder einen organischen Substituenten mit 1-10 Kohlenstoffatomen darstellt, in einer effektiven Menge, um die systemische Resistenz zu induzieren.

**17.** Verwendung einer Zusammensetzung gemäß den Ansprüchen 10-13 oder 16 zur Induktion einer systemischen Resistenz bei Pflanzen gegen pathologische Organismen.

**18.** Wäßrige Zusammensetzung, umfassend eine das Wachstum von Pathogenen modulierende Menge wenigstens einer Saponinverbindung sowie einer oder mehrerer Verbindungen der Formel

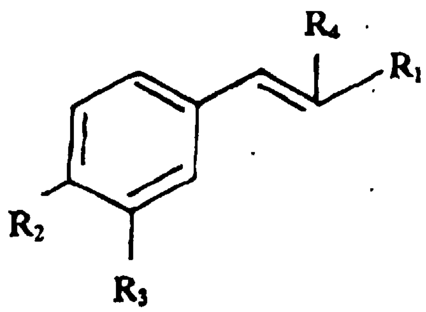

(2)

wobei $R_1$ -CHO darstellt, $R_2$ -H, -OH oder einen organischen Substituenten mit 1-10 Kohlenstoffatomen darstellt, $R_3$ -H, $-OCH_3$ oder einen organischen Substituenten mit 1-10 Kohlenstoffatomen darstellt und $R_4$ -H oder einen organischen Substituenten mit 1-10 Kohlenstoffatomen darstellt, in einem landwirtschaftlich verträglichen Träger, wobei die Zusammensetzung eine mittlere Krankheitsresistenz von 70% oder darüber gegen wenigstens einen pathogenen Organismus bewirkt, der eine oder mehrere Pflanzenoberflächen besiedelt.

**19.** Verwendung einer Zusammensetzung gemäß den Ansprüchen 10-13, 16 oder 18 zum Bewirken einer mittleren Krankheitsresistenz von 70% oder darüber gegen wenigstens einen pathogenen Organismus, der eine oder mehrere Pflanzenoberflächen besiedelt.

**20.** Verfahren zur Durchmusterung eines Pflanzenpathogens auf Empfindlichkeit gegenüber einer das Wachstum von Pilzen modulierenden Zubereitung, die wenigstens eine Saponinverbindung sowie wenigstens eine Verbindung der Formel

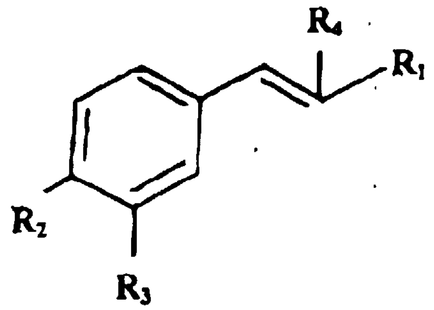

(2)

umfaßt, wobei $R_1$ -CHO darstellt, $R_2$ -H, -OH oder einen organischen Substituenten mit 1-10 Kohlenstoffatomen darstellt, $R_3$ -H, $-OCH_3$ oder einen organischen Substituenten mit 1-10 Kohlenstoffatomen darstellt und $R_4$ -H oder einen organischen Substituenten mit 1-10 Kohlenstoffatomen darstellt, wobei das Verfahren folgendes umfaßt:

In-Kontakt-Bringen des Pflanzenpathogens mit der das Wachstum von Pilzen modulierenden Zubereitung; und

Bestimmen der Wirksamkeit der Zubereitung als Fungizid.

**21.** Verfahren zur Bekämpfung des Wachstums pathologischer Organismen an einer Pflanze, wobei die Pflanzenoberfläche mit einer wäßrigen Zusammensetzung versehen wird, die eine oder mehrere Saponinverbindungen sowie wenigstens eine Verbindung gemäß der folgenden Formel

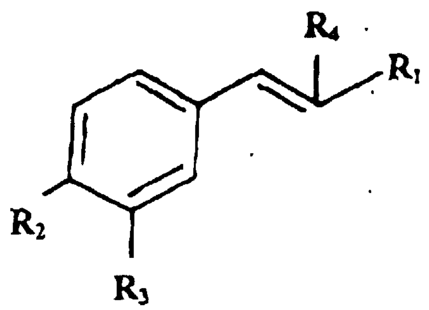

(2)

umfaßt, wobei $R_1$ -CHO darstellt, $R_2$ -H, -OH oder einen organischen Substituenten mit 1-10 Kohlenstoffatomen darstellt, $R_3$ -H, $-OCH_3$ oder einen organischen Substituenten mit 1-10 Kohlenstoffatomen darstellt und $R_4$ -H oder einen organischen Substituenten mit 1-10 Kohlenstoffatomen darstellt, wobei die Saponinverbindungen als Synergisten wirken und die Wirkung der Verbindung der Formel (2) bei der Bekämpfung des Wachstums pathologischer Organismen an einer Pflanze verstärken und eine Reduktion der Konzentration der Verbindung der Formel (2) in der Zubereitung ermöglichen im Vergleich zur Konzentration der Verbindung der Formel (2), wenn man sie ohne die Saponinverbindungen verwendet.

**22.** Verfahren gemäß Anspruch 21, wobei die Zubereitung eine wäßrige Zubereitung ist, die 0,01-50 g/l einer oder mehrerer Verbindungen gemäß Formel (2) von Anspruch 21 umfaßt.

**23.** Verfahren gemäß Anspruch 21 oder 22, wobei $R_1$ -CHO darstellt, $R_2$ und $R_3$ -H darstellen und $R_4$ $-(CH_2)_5-CH_3$ darstellt.

**24.** Wäßrige Zusammensetzung zur Verwendung bei einem Verfahren gemäß den Ansprüchen 21-23, umfassend eine oder mehrere Saponinverbindungen sowie 0,01-50 g/l einer oder mehrerer Verbindungen der Formel

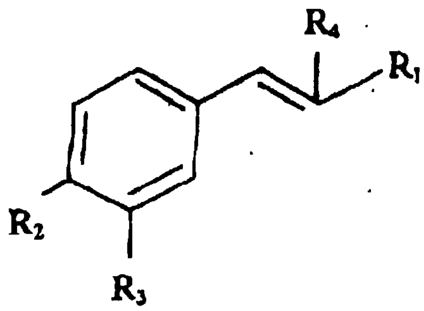

(2)

wobei $R_1$ -CHO darstellt, $R_2$ -H, -OH oder einen organischen Substituenten mit 1-10 Kohlenstoffatomen darstellt, $R_3$ -H, $-OCH_3$ oder einen organischen Substituenten mit 1-10 Kohlenstoffatomen darstellt und $R_4$ -H oder einen organischen Substituenten mit 1-10 Kohlenstoffatomen darstellt.

**25.** Zusammensetzung gemäß Anspruch 24, wobei $R_1$ -CHO darstellt, $R_2$ und $R_3$ -H darstellen und $R_4$ $-(CH_2)_5-CH_3$ darstellt.

**26.** Zusammensetzung gemäß Anspruch 24, wobei es sich bei wenigstens einer Verbindung um Zimtaldehyd oder Coniferylaldehyd handelt.

**Revendications**

**1.** Procédé pour contrôler la croissance d'organismes pathologiques sur une plante dans lequel la surface de la plante est munie d'une composition aqueuse comprenant une quantité contrôlant la croissance des organismes pathologiques efficace d'un ou plusieurs composés de type saponine et d'au moins un composé selon la formule suivante:

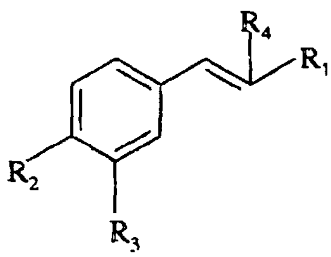

(2)

où $R_1$ représente -CHO, $R_2$ représente -H, -OH ou un substituant organique comprenant 1-10 atomes de carbone, $R_3$ représente -H, $-OCH_3$ ou un substituant organique comprenant 1-10 atomes de carbone et $R_4$ représente -H ou un substituant organique comprenant 1-10 atomes de carbone, où ladite quantité n'est pas phytotoxique pour ladite plante.

**2.** Procédé selon la revendication 1, dans lequel ladite plante est mise en contact avec une formulation aqueuse comprenant 0,01-50 g/l d'un ou plusieurs des composés selon la formule (2) de la revendication 1.

**3.** Procédé selon la revendication 1, dans lequel ladite formulation aqueuse comprend 0,025-3 % de saponine.

**4.** Procédé selon la revendication 1 ou 2, dans lequel $R_1$ représente -CHO, $R_2$ et $R_3$ représentent -H et $R_4$ représente $-(CH_2)_5-CH_3$.

**5.** Procédé selon l'une quelconque des revendications précédentes, dans lequel l'espèce de plante est une espèce de rosier, de vigne, de pommier, de pêcher, de gazon, de tomate, de paprika, de pin ou de coton.

**6.** Procédé selon l'une quelconque des revendications 1-5, dans lequel les organismes pathologiques comprennent au moins un organisme pathologique parmi les champignons, les bactéries, les nématodes, les algues, les insectes, les arachnides et les mollusques terrestres.

**7.** Procédé selon les revendications 5 et 6, dans lequel l'organisme pathologique est un champignon provoquant l'oïdum des cucurbitacées, la rouille, le botrytis, le chancre, la sclérotiniose, le chancre à *Phythium* et le chancre à *Rhizoctonia.*

**8.** Procédé selon l'une quelconque des revendications 1 à 6, dans lequel l'organisme pathologique est au moins un organisme pathologique parmi les pucerons, les cicadelles, les tordeuses, les nématodes des tiges et des bulbes, le phylloxera, les algues bleues, le carpocapse des pommes et des poires et le thrips.

**9.** Procédé selon l'une quelconque des revendications 1 à 3 et 5 à 8, dans lequel au moins l'un des composés est l'aldéhyde cinnamique ou le coniférylaldéhyde.

**10.** Composition aqueuse destinée à être utilisée dans un procédé selon l'une quelconque des revendications précédentes, comprenant un ou plusieurs composés de type saponine et 0,01-50 g/l d'un ou plusieurs composés de formule :

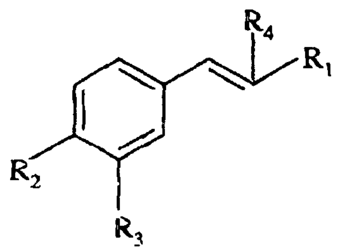

(2)

où $R_1$ représente -CHO, $R_2$ représente -H, -OH ou un substituant organique comprenant 1-10 atomes de carbone, $R_3$ représente -H, $-OCH_3$ ou un substituant organique comprenant 1-10 atomes de carbone et $R_4$ représente -H ou un substituant organique comprenant 1-10 atomes de carbone.

**11.** Composition selon la revendication 10, dans laquelle $R_1$ représente -CHO, $R_2$ et $R_3$ représentent -H et $R_4$ représente $-(CH_2)_5-CH_3$.

**12.** Composition selon la revendication 10 ou 11, dans laquelle ladite saponine est présente en une quantité de 0,025-3 %.

**13.** Composition selon la revendication 10, dans laquelle au moins un composé est l'aldéhyde cinnamique ou le coniférylaldéhyde.

**14.** Utilisation d'une composition selon l'une quelconque des revendications 10 à 13 pour contrôler la croissance d'organismes pathologiques sur une plante.

**15.** Procédé pour induire une résistance des plantes contre des organismes pathologiques dans lequel lesdites plantes sont mises en contact avec une quantité d'une combinaison d'au moins un composé de type saponine et d'au moins un composé de formule:

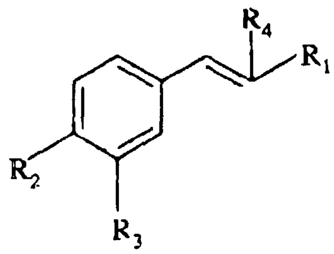

(2)

où $R_1$ représente -CHO, $R_2$ représente -H, -OH ou un substituant organique comprenant 1-10 atomes de carbone, $R_3$ représente -H, $-OCH_3$ ou un substituant organique comprenant 1-10 atomes de carbone et $R_4$ représente -H ou un substituant organique comprenant 1-10 atomes de carbone, où ladite quantité est suffisante pour induire une résistance systémique auxdits organismes pathologiques.

**16.** Composition aqueuse destinée à être utilisée dans un procédé selon la revendication 15, comprenant au moins un composé de type saponine et au moins un composé de formule:

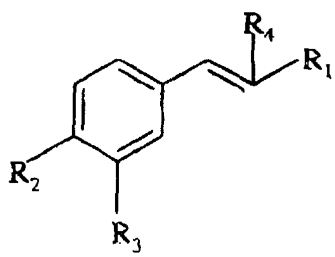

(2)

où $R_1$ représente -CHO, $R_2$ représente -H, -OH ou un substituant organique comprenant 1-10 atomes de carbone, $R_3$ représente -H, $-OCH_3$ ou un substituant organique comprenant 1-10 atomes de carbone et $R_4$ représente -H ou un substituant organique comprenant 1-10 atomes de carbone en une quantité efficace pour induire ladite résistance systémique.

**17.** Utilisation d'une composition selon les revendications 10-13 ou 16 pour induire une résistance systémique dans des plantes contre des organismes pathologiques.

**18.** Composition aqueuse comprenant une quantité modulant la croissance des agents pathogènes d'au moins un composé de type saponine et d'un ou plusieurs composés de formule:

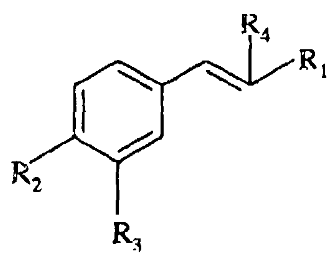

(2)

où $R_1$ représente -CHO, $R_2$ représente -H, -OH ou un substituant organique comprenant 1-10 atomes de carbone, $R_3$ représente -H, $-OCH_3$ ou un substituant organique comprenant 1-10 atomes de carbone et $R_4$ représente -H ou un substituant organique comprenant 1-10 atomes de carbone, dans un support compatible du point de vue agricole, où ladite composition confère une résistance moyenne aux maladies de 70% ou plus contre au moins un organisme pathogène colonisant une ou plusieurs surfaces des plantes.

**19.** Utilisation d'une composition selon les revendications 10-13, 16 ou 18 pour conférer une résistance moyenne aux maladies de 70 % ou plus contre au moins un organisme pathogène colonisant une ou plusieurs surfaces des plantes.

**20.** Procédé de criblage concernant la sensibilité d'un agent pathogène de plantes à une formulation modulant la croissance fongique comprenant au moins un composé de type saponine et au moins un composé de formule:

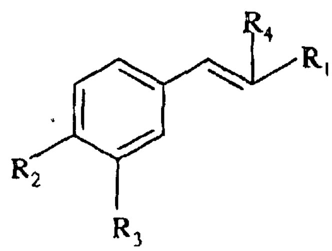

(2)

où $R_1$ représente -CHO, $R_2$ représente -H, -OH ou un substituant organique comprenant 1-10 atomes de carbone, $R_3$ représente -H, $-OCH_3$ ou un substituant organique comprenant 1-10 atomes de carbone et $R_4$ représente -H ou un substituant organique comprenant 1-10 atomes de carbone, ledit procédé comprenant:

la mise en contact dudit agent pathogène de plantes avec ladite formulation modulant la croissance fongique ; et la détermination de l'efficacité de ladite formulation comme agent pathogène des champignons.

**21.** Procédé pour contrôler la croissance d'organismes pathologiques sur une plante dans lequel la surface de la plante est munie d'une composition aqueuse comprenant un ou plusieurs composés de type saponine et au moins un composé selon la formule suivante:

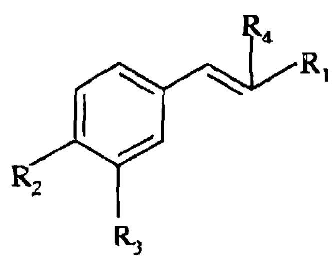

(2)

où $R_1$ représente -CHO, $R_2$ représente -H, -OH ou un substituant organique comprenant 1-10 atomes de carbone, $R_3$ représente -H, $-OCH_3$ ou un substituant organique comprenant 1-10 atomes de carbone et $R_4$ représente -H ou un substituant organique comprenant 1-10 atomes de carbone, où lesdits composés de type saponine sont des agents de synergie pour augmenter l'effet dudit composé de formule (2) sur le contrôle de la croissance d'organismes pathologiques sur une plante et permettent une réduction de la concentration dudit composé de formule (2) dans ladite formulation par rapport à la concentration dudit composé de formule (2) quand il est utilisé sans lesdits composés de type saponine.

**22.** Procédé selon la revendication 21, dans lequel ladite formulation est une formulation aqueuse comprenant 0,01-50 g/l d'un ou plusieurs des composés selon la formule (2) de la revendication 21.

**23.** Procédé selon la revendication 21 ou 22, dans lequel $R_1$ représente -CHO, $R_2$ et $R_3$ représentent -H et $R_4$ représente $-(CH_2)_5-CH_3$.

**24.** Composition aqueuse destinée à être utilisée dans un procédé selon les revendications 21-23 comprenant un ou plusieurs composés du type saponine et 0,01-50 g/l d'un ou plusieurs composés de formule:

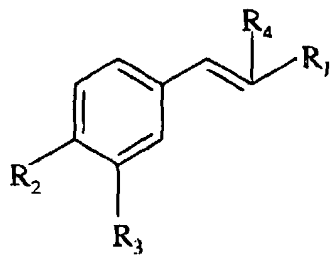

(2)

où $R_1$ représente -CHO, $R_2$ représente -H, -OH ou un substituant organique comprenant 1-10 atomes de carbone, $R_3$ représente -H, $-OCH_3$ ou un substituant organique comprenant 1-10 atomes de carbone et $R_4$ représente -H ou un substituant organique comprenant 1-10 atomes de carbone.

**25.** Composition selon la revendication 24, dans laquelle $R_1$ représente -CHO, $R_2$ et $R_3$ représentent -H et $R_4$ représente $-(CH_2)_5-CH_3$.

**26.** Composition selon la revendication 24, dans laquelle au moins un composé est l'aldéhyde cinnamique ou le coniférylaldéhyde.